# EUROPEAN PATENT APPLICATION

(11) **EP 4 202 319 A1**
(43) Date of publication of application: **28.06.2023**
(21) Application number: 21858294.8
(22) Date of filing: 17.08.2021
(51) Int. Cl.: F25B 9/14, H01F 6/04, C09K 5/14, F28D 20/00, G01N 24/00, A61B 5/055

(54) **COLD STORAGE MATERIAL PARTICLES, COLD STORAGE DEVICE, REFRIGERATING MACHINE, CRYOPUMP, SUPERCONDUCTING MAGNET, NUCLEAR MAGNETIC RESONANCE IMAGING APPARATUS, NUCLEAR MAGNETIC RESONANCE APPARATUS, MAGNETIC FIELD APPLICATION TYPE SINGLE CRYSTAL PULLING APPARATUS, AND MEHOD FOR PRODUCING COLD STORAGE MATERIAL PARTICLES**

(30) Priority: 18.08.2020 JP 2020137850; 25.09.2020 JP 2020160699
(71) Applicant: KABUSHIKI KAISHA TOSHIBA, Minato-ku Tokyo 105-0023 (JP); Toshiba Materials Co., Ltd., Isogo-Ku Yokohama-Shi Kanagawa 235-0032 (JP)
(72) Inventor: YAMASHITA, Tomohiro, Tokyo 105-0023 (JP); EGUCHI, Tomoko, Tokyo 105-0023 (JP); KUBOKI, Takashi, Tokyo 105-0023 (JP); USUI, Daichi, Yokohama-shi, Kanagawa 235-0032 (JP); KAWAMOTO, Takahiro, Yokohama-shi, Kanagawa 235-0032 (JP)
(74) Representative: Henkel & Partner mbB
(86) International application number: PCT/JP2021/030014
(87) International publication number: WO 2022/039150

(57) **Abstract**

COLD STORAGE MATERIAL PARTICLES, COLD STORAGE DEVICE, REFRIGERATING MACHINE, CRYOPUMP, SUPERCONDUCTING MAGNET, NUCLEAR MAGNETIC RESONANCE IMAGING APPARATUS, NUCLEAR MAGNETIC RESONANCE APPARATUS, MAGNETIC FIELD APPLICATION TYPE SINGLE CRYSTAL PULLING APPARATUS, AND MEHOD FOR PRODUCING COLD STORAGE MATERIAL PARTICLES

## Description

### TECHNICAL FIELD

Embodiments of the present invention relate to a cold storage material particle, a cold storage device, a refrigerator, a cryopump, a superconducting magnet, a nuclear magnetic resonance imaging apparatus, a nuclear magnetic resonance apparatus, a magnetic field application type single crystal pulling apparatus, and a method for producing a cold storage material particle.

### BACKGROUND ART

In a cryogenic refrigerator used for cooling a superconducting device and forming ultrahigh vacuum, cold storage material particles containing a cold storage substance having high volume specific heat in a low temperature region are used. Here, specific heat per unit volume is defined as volume specific heat. As the cold storage substance, for example, metals such as lead (Pb) and bismuth (Bi), rare earth compounds such as HoCu₂ and Er₃Ni, oxides such as Ag₂O and Cu₂O, and oxysulfides such as Gd₂O₂S are used.

In a cryogenic refrigerator, a cold storage device is filled with a plurality of cold storage material particles. For example, cold is generated by performing heat exchange between the cold storage material particles and helium gas passing through the cold storage device. For example, in a superconducting nuclear magnetic resonance apparatus (MRI apparatus), a cryopump, and the like, a refrigerator using a refrigeration cycle such as a Gifford-McMahon (GM) system, a Stirling system, or a pulse tube system is used.

In addition, a high-performance refrigerator is also required for a magnetically levitated train in order to generate a magnetic force using a superconducting magnet. In addition, in a semiconductor manufacturing apparatus, a cryopump is used to make a chamber for forming a thin film on a wafer ultrahigh vacuum. Furthermore, in recent years, high-performance refrigerators have also been used in a superconducting magnetic energy storage (SMES), a magnetic field application type single crystal pulling apparatus that manufactures high-quality silicon wafers, and the like. Further, development and practical application of a pulse tube refrigerator, which is expected to have high reliability, are also actively promoted.

In such a refrigerator, a working medium such as compressed helium (He) gas flows in one direction in a cold storage device filled with the cold storage material particles, and the thermal energy is supplied to a cold storage material. Then, the expanded working medium flows in the opposite direction in the cold storage device, and receives thermal energy from the cold storage material particles. As a recuperating effect in such a process is improved, thermal efficiency in a working medium cycle is improved, and a lower temperature can be realized.

A cold storage container of the refrigerator is filled with the cold storage material particles, and refrigeration capacity in a cryogenic temperature region is exhibited by heat exchange with the helium (He) gas flowing in as the working medium. In this case, the cold storage material particles receive pressure vibration caused by the high-pressure helium gas acting during the operation of the refrigerator, stress caused by the high-pressure helium gas, and an impact force caused by the high-pressure helium gas. In the case of a GM refrigerator, stress due to a reciprocating motion of a displacer (working medium compression piston) further acts on the cold storage material particles. In addition, at the time of starting the refrigerator, the temperature drops from around room temperature to a cryogenic temperature around 4K in a short time, so that a large thermal shock acts on the cold storage material particles.

In this manner, the cold storage material particles are broken and pulverized by pressure vibration and various stresses acting during operation of the refrigerator. It is a problem that the generated fine powder damages a component such as a seal portion of the refrigerator to significantly lower the refrigeration capacity. In order to maintain high refrigeration capacity of the refrigerator for a long period of time, that is, in order to improve long-term reliability of the refrigerator, the cold storage material particles filling the cold storage device of the refrigerator are required to have excellent characteristics such as, for example, high volume specific heat, high mechanical strength, high thermal conductivity, and high heat transfer.

### CITATION LIST

### PATENT LITERATURE

Patent Literature 1: JP 2017-58079 A
Patent Literature 2: JP 2010-64946 A
Patent Literature 3: JP 2003-213252 A
Patent Literature 4: WO 2018/025581 A

### SUMMARY OF THE INVENTION

### PROBLEM TO BE SOLVED BY THE INVENTION

An object of the present invention is to provide a cold storage material particle having high mechanical strength and a method for producing the same.

### MEANS FOR SOLVING PROBLEM

A cold storage material particle of an embodiment includes at least one first element selected from the group consisting of a rare earth element, silver (Ag), and copper (Cu) and a second element that is different from the first element and forms a multivalent metal ion in an aqueous solution, in which an atomic concentration of the second element is 0.001 atomic% or more and 60 atomic% or less, and a maximum value of volume specific heat at a temperature of 20K or less is 0.3 J/cm³·K or more.

### BRIEF DESCRIPTION OF THE DRAWINGS

FIG. 1 is an explanatory view of a cold storage material particle according to a first embodiment.
FIG. 2 is an explanatory view of a cold storage material particle according to a second embodiment.
FIG. 3 is an explanatory view of a cold storage material particle according to a third embodiment.
FIG. 4 is a schematic cross-sectional view illustrating a configuration of a main part of a GM refrigerator which is an example of a refrigerator according to a fourth embodiment.
FIG. 5 is a cross-sectional view illustrating a schematic configuration of a cryopump according to a fifth embodiment.
FIG. 6 is a perspective view illustrating a schematic configuration of a superconducting magnet of a sixth embodiment.
FIG. 7 is a cross-sectional view illustrating a schematic configuration of a nuclear magnetic resonance imaging apparatus of a seventh embodiment.
FIG. 8 is a cross-sectional view illustrating a schematic configuration of a nuclear magnetic resonance apparatus of an eighth embodiment.
FIG. 9 is a perspective view illustrating a schematic configuration of a magnetic field application type single crystal pulling apparatus according to a ninth embodiment.

### EMDODIMENT(S) FOR CARRYING OUT THE INVENTION

Hereinafter, embodiments of the present invention will be described with reference to the drawings. In the following description, the same or similar members and the like are denoted by the same reference numerals, and the description of the members and the like once described may be appropriately omitted.

In the present specification, a cryogenic temperature means, for example, a temperature range in which a superconducting phenomenon can be industrially useful. For example, the temperature range is a temperature range of 20K or less.

### (First Embodiment)

A cold storage material particle of a first embodiment includes at least one first element selected from the group consisting of a rare earth element, silver (Ag), and copper (Cu), and a second element that is different from the first element and forms a multivalent metal ion in an aqueous solution. An atomic concentration of the second element is 0.001 atom% or more and 60 atom% or less. In addition, a maximum value of volume specific heat at a temperature of 20K or less is 0.3 J/cm³·K or more.

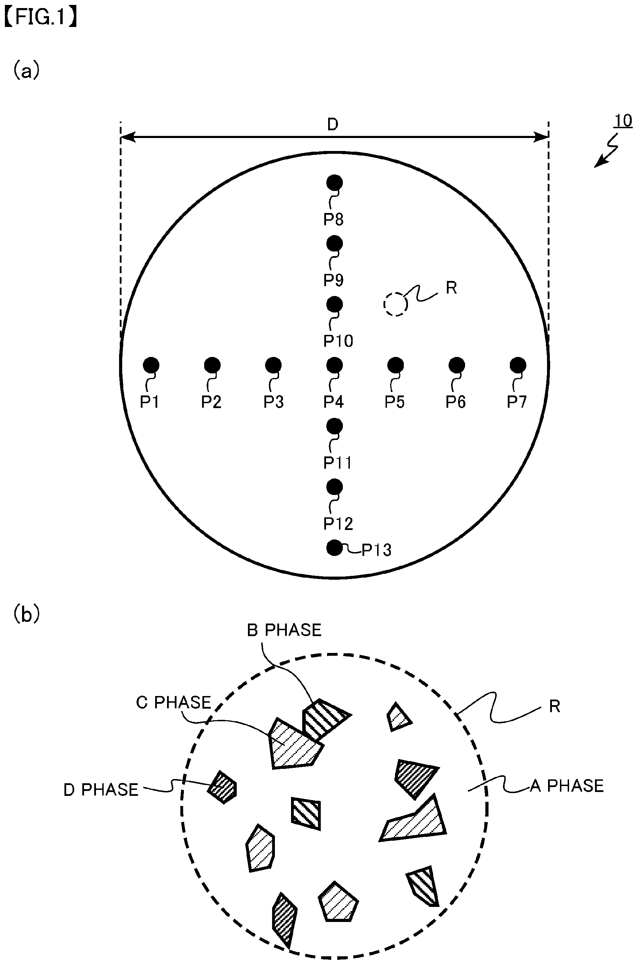

FIG. 1 is an explanatory view of the cold storage material particle according to the first embodiment. FIG. 1(a) is a schematic cross-sectional view of the cold storage material particle. FIG. 1(b) is a schematic cross-sectional view in which a part of the cold storage material particle is enlarged. FIG. 1(b) is a schematic cross-sectional view of a region R in FIG. 1(a), for example.

A cold storage material particle 10 of the first embodiment is used, for example, in a refrigerator that achieves a cryogenic temperature of 5K or less.

The shape of the cold storage material particle 10 is, for example, spherical. FIG. 1(a) illustrates a case where the cold storage material particle 10 is a true sphere. FIG. 1(a) is a cross section passing through a central portion of the cold storage material particle 10. A particle diameter (D in FIG. 1(a)) of the cold storage material particle is, for example, 50 um or more and 3 mm or less. An aspect ratio of the cold storage material particle 10 is, for example, 5 or less. The aspect ratio of the cold storage material particle 10 is a ratio of a major axis to a minor axis of the cold storage material particle 10.

The particle diameter D of the cold storage material particle 10 is an equivalent circle diameter. The equivalent circle diameter is a diameter of a perfect circle corresponding to an area of a figure observed in an image such as an optical microscope image or a scanning electron microscope image (SEM image). The particle diameter D of the cold storage material particle 10 can be obtained, for example, by image analysis of an optical microscope image or an SEM image.

In the cold storage material particle 10, the maximum value of volume specific heat at a temperature of 20K or less is 0.3 J/cm³·K or more. The cold storage material particle 10 contains a cold storage substance having a maximum value of volume specific heat of 0.3 J/cm³·K or more at a temperature of 20K or less.

The cold storage material particle 10 contains a cold storage element. The cold storage element is at least one element selected from the group consisting of a rare earth element, silver (Ag), and copper (Cu). The rare earth element is at least one element selected from the group consisting of scandium (Sc), yttrium (Y), lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), and lutetium (Lu).

The cold storage element is one of elements constituting the cold storage substance. The cold storage element is an example of the first element.

The cold storage material particle 10 contains an additive metal element. The additive metal element is a metal element that forms a multivalent metal ion in an aqueous solution. The additive metal element is, for example, at least one element selected from the group consisting of calcium (Ca), magnesium (Mg), beryllium (Be), strontium (Sr), barium (Ba), radium (Ra), manganese (Mn), aluminum (Al), iron (Fe), copper (Cu), nickel (Ni), and cobalt (Co).

The additive metal element is an example of the second element. For example, two or more kinds of additive metal elements may be contained in the cold storage material particle 10.

The atomic concentration of the additive metal element in the cold storage material particle 10 is 0.001 atom% or more and 60 atom% or less. When the cold storage material particle 10 contains two or more kinds of additive metal elements, the total of the atomic concentrations of the respective additive metal elements is taken as the atomic concentration of the additive metal element of the cold storage material particle 10.

The atomic concentration of the additive metal element is smaller than the atomic concentration of the cold storage element, for example. The atomic concentration of the additive metal element is larger than the atomic concentration of the cold storage element, for example.

The detection of the element contained in the cold storage material particle 10 and the measurement of the atomic concentration of the element can be performed using, for example, energy dispersive X-ray spectroscopy (EDX) or wavelength dispersive X-ray spectroscopy (WDX).

The atomic concentration of the additive metal element in the cold storage material particle 10 is, for example, an average value of the atomic concentrations measured at 13 measurement spots P1 to P13 as illustrated in FIG. 1(a). The atomic concentration of the added metal is an atomic concentration having the amount of all atoms measured by WDX or EDX as a denominator. The measurement spot is, for example, ϕ 20 µm.

Note that the detection of the element contained in the cold storage material particle 10 and the measurement of the atomic concentration of the element can also be performed, for example, by dissolving the cold storage material particle 10 in a liquid and using inductively coupled plasma atomic emission spectrometry (Inductively Coupled Plasma Atomic Emission Spectroscopy: ICP-AES).

The cold storage substance contained in the cold storage material particle 10 contains, for example, an oxysulfide. The cold storage substance contains, for example, oxysulfide as a main component. When the cold storage substance contains oxysulfide, the cold storage material particle 10 contains oxygen (O) and sulfur (S).

The oxysulfide contained in the cold storage substance contains, for example, gadolinium (Gd). The oxysulfide contained in the cold storage substance is, for example, gadolinium oxysulfide. The oxysulfide contained in the cold storage substance is, for example, Gd₂O₂S.

When the oxysulfide contained in the cold storage substance is gadolinium oxysulfide, the cold storage element is gadolinium (Gd).

The cold storage substance contained in the cold storage material particle 10 can be identified using, for example, powder X-ray diffraction (XRD).

The cold storage substance contained in the cold storage material particle 10 contains, for example, a rare earth compound as a main component. A rare earth compound contained in the cold storage substance is, for example, HoCu₂ or Er₃Ni.

When the cold storage substance contains HoCu₂, the cold storage elements are holmium (Ho) and copper (Cu). When the cold storage substance contains Er₃Ni, the cold storage element is erbium (Er).

The cold storage substance contained in the cold storage material particle 10 contains, for example, an oxide. The cold storage substance contains, for example, an oxide as a main component. When the cold storage substance contains an oxide, the cold storage material particle 10 contains oxygen (O).

The oxide contained in the cold storage substance contains, for example, at least one of silver (Ag) and copper (Cu). The oxide contained in the cold storage substance is, for example, silver oxide or copper oxide. The oxide contained in the cold storage substance is, for example, Ag₂O or Cu₂O.

When the oxide contained in the cold storage substance is silver oxide, the cold storage element is silver (Ag). When the oxide contained in the cold storage substance is copper oxide, the cold storage element is copper (Cu).

A composition analysis of the cold storage substance can be performed using, for example, EDX or WDX. The cold storage substance can be identified using, for example, a powder X-ray diffraction method.

FIG. 1(b) is a schematic cross-sectional view in which a part of the cold storage material particle is enlarged. FIG. 1(b) is a schematic cross-sectional view of a region R in FIG. 1(a), for example.

As illustrated in FIG. 1(b), the cold storage material particle 10 of the first embodiment includes four phases of an A phase, a B phase, a C phase, and a D phase. The A phase, the B phase, the C phase, and the D phase are different phases. The A phase, the B phase, the C phase, and the D phase are examples of a first phase, a second phase, a third phase, and a fourth phase, respectively. The two phases being different means that at least the chemical compositions of the two phases are different.

The A phase, the B phase, the C phase, and the D phase are, for example, crystal phases. The A-phase, the B-phase, the C-phase, or the D-phase may be, for example, an amorphous phase.

The phase A is a cold storage substance. The A phase contains a cold storage element. The A phase is, for example, gadolinium oxysulfide. The A phase is, for example, Gd₂O₂S.

When the A phase is gadolinium oxysulfide, the cold storage element is gadolinium (Gd). The A phase contains gadolinium (Gd), sulfur (S), and oxygen (O).

The cold storage material particle 10 contains, for example, two or more different additive metal elements. The cold storage material particle 10 contains a first additive metal element and a second additive metal element different from the first additive metal element. The first additive metal element is an example of an element α. The second additive metal element is an example of an element β.

The B phase contains the first additive metal element and the second additive metal element. The B phase contains two or more different elements including the first additive metal element and the second additive metal element. An atomic concentration of the additive metal element in the B phase is larger than an atomic concentration of the additive metal element in the A phase.

The B phase is, for example, an oxide containing calcium (Ca) and aluminum (Al). The B phase is, for example, a compound containing calcium (Ca), aluminum (Al), and oxygen (O). The B phase is, for example, CaO·6Al₂O₃.

When the B phase is an oxide containing calcium (Ca) and aluminum (Al), the first additive metal element is calcium (Ca), and the second additive metal element is aluminum (Al).

The B phase further contains, for example, a cold storage element. The B-phase contains, for example, gadolinium (Gd). In this case, the B phase contains calcium (Ca), aluminum (Al), gadolinium (Gd), and oxygen (O).

The C phase contains the second additive metal element.

The C phase is, for example, aluminum oxide. The C phase is, for example, Al₂O₃.

When the C phase is aluminum oxide, the C phase contains aluminum (Al) and oxygen (O).

The D-phase contains the cold storage element and the second additive metal element. The D-phase is, for example, an oxide containing gadolinium (Gd) and aluminum (Al). The D-phase is, for example, a compound containing gadolinium (Gd), aluminum (Al), and oxygen (O). The D-phase is, for example, GdAlO₃.

In a cross section of the cold storage material particle 10, for example, the A phase surrounds the B phase, the C phase, and the D phase. In the cross section of the cold storage material particle 10, a proportion of an area occupied by the A phase containing the cold storage element is, for example, larger than a proportion of an area occupied by other phases.

In the cross section of the cold storage material particle 10, a proportion of an area occupied by the phase B containing the additive metal element is, for example, 0.001% or more and 75% or less.

In the cross section of the cold storage material particle 10, the proportion of the area occupied by the A phase, the proportion of the area occupied by the B phase, the proportion of the area occupied by the C phase, and the proportion of the area occupied by the D phase can be obtained, for example, by image analysis of an optical microscope image or a scanning electron microscope image (SEM image).

For example, by photographing a reflected electron image of a cross section of the cold storage material particle 10 with a scanning electron microscope (SEM), phases can be distinguished from a difference in density of the image. In addition, for example, by performing composition analysis at points in each phase by EDX or WDX, the types and element concentrations of elements contained in each phase can be found. For example, the compound contained in each phase can be obtained by considering the results of XRD in addition to the results of EDX or WDX.

In the image analysis of the scanning electron microscope image (SEM image), for example, an image of a reflected electron image is used. For example, ImageJ is used as the image analysis software. A region corresponding to each phase can be extracted from the image from the brightness of the reflected electron image. When a region corresponding to each phase is extracted, for example, brightness is binarized. For example, the extracted region and the original image are compared with the naked eye, and in a case where it is clear that the grain boundary or the inside of the grain is not appropriately extracted, the area of each phase is evaluated after the image is manually corrected. As software for correcting an image, for example, software "paint" included in Windows (registered trademark) 10 as a standard is used.

In the cross section of the cold storage material particle 10, the area per phase B containing the additive metal element is, for example, 0.001 µm² or more and 6000 µm² or less. The area per one of the B phases containing the additive metal element is, for example, a median of areas of a plurality of B phases.

In the cross section of the cold storage material particle 10, a particle diameter of one B phase containing the additive metal element is, for example, 0.1 um or more and 100 um or less. Here, the particle diameter is, for example, a major axis of the B phase. The major axis of the B phase is the maximum length in the length between any two points on the outer periphery of the B phase. The particle diameter of each of the B phases containing the additive metal element is, for example, a median of particle diameters of a plurality of B phases.

In the cross section of the cold storage material particle 10, the area and particle diameter of the B phase containing the additive metal element can be determined, for example, by image analysis of an optical microscope image or a scanning electron microscope image.

Next, an example of a method for producing the cold storage material particle 10 of the first embodiment will be described.

A method for producing a cold storage material particle according to a first embodiment includes mixing powder containing at least one first element selected from the group consisting of a rare earth element, silver (Ag), and copper (Cu) with an alginic acid aqueous solution to form a slurry, discharging the slurry in the form of droplets into a gelling solution containing a second element that forms multivalent metal ions, holding the slurry in the gelling solution to form gelled particles, and sintering the particles.

First, a raw material powder of the cold storage substance is added to and mixed with an alginic acid aqueous solution to prepare a slurry. The cold storage substance includes a cold storage element. For mixing the raw material powder of the cold storage substance and the alginic acid aqueous solution, for example, a ball mill is used.

A mixing time is, for example, 1 hour or more and 1 week or less. By changing the mixing time, for example, it is possible to change a proportion of the second phase in contact with the third phase and a proportion of the second phase in contact with the fourth phase in a cross section of the cold storage material particle to be manufactured.

Next, the slurry is discharged in the form of droplets to the gelling solution containing the second element that forms multivalent metal ions. The prepared slurry is dropped into a gelling solution to gel the slurry. As an apparatus for dropping the slurry into the gelling solution, for example, a dropper, a burette, a pipette, a syringe, a dispenser, or an inkjet is used.

The viscosity of the slurry suitable for discharge varies depending on the apparatus used for discharge. For example, the viscosity is 0.1 mPa·s or more and 1000000 mPa·s in the case of syringe ejection, 50 mPa·s or more and 300000 mPa·s or less in the case of a dispenser, and 1 mPa·s or more and 1000 mPa·s or less in the case of inkjet. The viscosity of the slurry is appropriately adjusted according to the apparatus used for discharge.

The production speed of the particles varies depending on the discharge method. For example, in the case of syringe ejection, the production speed of particles is about 1 particle/second, but it is about 1 particle/second to 400 particles/second by using a dispenser, and it is about 500 particles/second to 2000 particles/second by inkjet. The production speed further increases by adding the same nozzle.

When a syringe is used, the diameter of the discharge port of the syringe is, for example, 50 um or more and 3000 um or less. A distance from a tip of the syringe to a liquid level of the gelling solution is, for example, 1 mm or more and 1000 mm or less. By changing a diameter of the discharge port of the syringe and the distance from the tip of the syringe to the liquid level of the gelling solution, for example, the particle diameter and the aspect ratio of the particles can be changed.

When the dispenser is used for dispensing, any of an air pulse dispenser, a plunger dispenser, and a piezo dispenser may be used as the device. At the discharge port, the slurry is discharged drop by drop by a rod driven up and down by high-pressure air in the air pulse dispenser. In the case of a piezo dispenser, the slurry is discharged drop by drop by a pressure wave generated by a volume change of a rod or piezo driven up and down by a piezo element. Here, the particle diameter can be changed by changing the diameter of the discharge port of the nozzle, that is, the nozzle diameter. The nozzle diameter is, for example, 50 um or more and 3000 um or less.

As described above, the slurry is discharged by driving the rod up and down. Therefore, the particle production speed changes depending on the time during which the rod is driven up and down. The time during which the rod is driven is, for example, 2.5 ms or more and 100 ms or less per cycle.

By changing the distance from the nozzle tip of the dispenser to the liquid level of the gelling solution, for example, the particle diameter and the aspect ratio of the particles can be changed. The distance from the tip of the nozzle to the liquid level of the gelling solution is, for example, 0.1 mm or more and 1000 mm or less.

The inkjet is roughly divided into a continuous type and an on-demand type as an ejection method, but any type of ejection method may be used. Further, the on-demand type is divided into three types of a piezo type, a thermal type, and a valve type, but any type may be used.

In the continuous type, a pressure wave is applied to a liquid column ejected by pressurization of a flow path to promote particle-forming of the liquid column. The pressure wave is applied by a pump, a piezoelectric element, or the like. Meanwhile, in the on-demand type, the droplets are discharged one by one, but the method of discharging the droplets is different depending on the method.

In the piezo method, a drop is ejected every drop by a pressure wave generated from a rapid volume change of the piezo element caused by applying a voltage to the piezo element. In the thermal method, air bubbles are generated in the liquid to be discharged by heater heating, so that the liquid droplets are discharged one by one. In the valve type, the lid of the nozzle outlet is opened and closed by a solenoid to discharge droplets one by one.

In the thermal method, moisture is evaporated by heating the slurry, and there is a high possibility that raw material powder is clogged in a flow path and discharge is inhibited. Therefore, when the on-demand type inkjet is used, the piezo method and the valve method are more preferable than the thermal method.

The diameter of the discharge port of the inkjet nozzle is, for example, 50 um or more and 3000 um or less. The distance from the tip of the nozzle to the liquid level of the gelling solution is, for example, 0.1 mm or more and 1000 mm or less. By changing the diameter of the discharge port of the nozzle and the distance from the tip of the syringe to the liquid level of the gelling solution, for example, the particle diameter and the aspect ratio of the particles can be changed.

Also in the ink jet, when the slurry viscosity changes, the amount of slurry discharged from the discharge port per unit time changes, and thus the particle diameter of the particles changes. Therefore, by adjusting the nozzle diameter according to the slurry viscosity, the particle diameter of the particles is adjusted to 50 um or more and 3 mm or less.

In the continuous type inkjet, the production speed of the particles can be changed by changing the waveform of the pressure wave applied to a liquid column ejected by flow path pressurization. In the on-demand type piezoelectric ink jet, the production speed of the particles can be changed by changing the frequency of the volume change of the piezoelectric element. In the on-demand thermal ink jet, the production speed of the particle can be changed by changing the heater heating frequency. Further, in the on-demand valve type inkjet, the production speed of the particles can be changed by changing the frequency of valve opening and closing. The frequency in each inkjet is, for example, 500 Hz or more and 2000 Hz or less. When the frequency is 500 Hz or more and 2000 Hz or less, the particle production speed is 500 particles/second or more and 2000 particles/second or less.

The slurry dropped on the gelling solution by a dropper, a burette, a pipette, a syringe, a dispenser, an inkjet, or the like is held in the gelling solution to be gelled. By gelling the slurry, spherical particles containing the raw material powder of the cold storage substance are formed.

The gelling solution contains an additive metal element that has become a multivalent metal ion. Gelation proceeds by a crosslinking reaction by a multivalent metal ion. As a retention time of the slurry in the gelling solution elapses, the additive metal element penetrates from the outer edge toward the center of the particle. A distribution of the additive metal element in the particles can be controlled by changing the time for holding the slurry in the gelling solution.

The retention time of the slurry in the gelling solution is, for example, 10 minutes or more and 48 hours or less. Hereinafter, the retention time of the slurry in the gelling solution is also simply referred to as a gelation time. By setting the retention time of the slurry in the gelling solution to 10 minutes or more, it is possible to obtain gelled particles in which the dripped slurry is gelled.

After the particles are formed by the gelation, the particles are cleaned with pure water. By cleaning the particles, the additive metal element adsorbed on the surfaces of the particles is removed.

After cleaning the particles, the particles are dried. After drying the particles, the particles are sintered to increase the mechanical strength of the particles and the cold storage substance density in the particles. When forming an oxysulfide cold storage substance, for example, the particles are sulfurized before sintering the particles.

When the particles are sulfurized, heat treatment is performed in a sulfurization atmosphere. The sulfurization atmosphere contains, for example, a gas containing a sulfur atom having a negative oxidation number, such as hydrogen sulfide (H₂S), carbon sulfide (CS₂), or methanethiol (CH₃SH). A heat treatment temperature is, for example, 400°C or more and 700°C or less. A heat treatment time is, for example, 1 hour or more and 8 hours or less.

The heat treatment for sintering the particles is performed, for example, in an atmosphere of an inert gas. The heat treatment temperature is, for example, 1000°C or more and 2000°C or less. The heat treatment temperature is, for example, 1100°C or more and 1700°C or less. The heat treatment time is, for example, 1 hour or more and 48 hours or less.

When the particles are sintered, for example, a first phase containing a cold storage element and a second phase containing an additive metal element are formed. In addition, by adding two or more kinds of additive metal elements, for example, a third phase containing the additive metal element or a fourth phase containing the cold storage element and the additive metal element can be formed.

The second phase, the third phase, or the fourth phase can be formed, for example, by appropriately managing the profile of the heat treatment temperature at the time of sintering the particles or the temperature rise and fall of the heat treatment.

In addition, for example, by changing the heat treatment conditions for sulfurization and sintering of the particles, it is possible to change the area and particle diameter per second phase in the cross section of the particles.

The cold storage substance is, for example, silver oxide, copper oxide, or rare earth oxysulfide. The rare earth oxysulfide is, for example, gadolinium oxysulfide.

The alginic acid aqueous solution is, for example, a sodium alginate aqueous solution, an ammonium alginate aqueous solution, or a potassium alginate aqueous solution.

Examples of the gelling solution include a calcium lactate aqueous solution, a calcium chloride aqueous solution, a manganese (II) chloride aqueous solution, a magnesium sulfate aqueous solution, a beryllium sulfate aqueous solution, a strontium nitrate aqueous solution, a barium chloride aqueous solution, a barium hydroxide aqueous solution, an aluminum chloride aqueous solution, an aluminum nitrate aqueous solution, an aluminum lactate aqueous solution, an iron (II) chloride aqueous solution, an iron (III) chloride aqueous solution, a copper (II) chloride aqueous solution, a nickel (II) chloride aqueous solution, and a cobalt (II) chloride aqueous solution.

A combination of the cold storage substance, the alginic acid aqueous solution, and the gelling solution is arbitrary. However, in a case where the cold storage substance is silver oxide, when a calcium chloride aqueous solution, a manganese (II) chloride aqueous solution, a barium chloride aqueous solution, an aluminum chloride aqueous solution, an iron (II) chloride aqueous solution, an iron (III) chloride aqueous solution, a copper (II) chloride aqueous solution, a nickel (II) chloride aqueous solution, or a cobalt (II) chloride aqueous solution is combined as a gelling solution, silver chloride is generated. Thus, the above combinations are excluded.

The cold storage material particle 10 of the first embodiment can be manufactured by the above producing method.

For example, the atomic concentration distribution of the additive metal element in the particles can be controlled to an arbitrary distribution by controlling the gelation time. For example, by controlling the gelation time, the additive metal element penetrates into the central part of the particle, and the additive metal element can be almost uniformly distributed in the particle.

In addition, by controlling the amount of the raw material powder of the cold storage substance, the atomic concentration of the additive metal element in the gelling solution, and the sintering conditions of the particles, the chemical composition of the first phase and the second phase of the cold storage material particle 10, an area ratio of the first phase and the second phase in the particles in the cross section of the cold storage material particle 10, and the areas of the first phase and the second phase in the cross section of the cold storage material particle 10 can be adjusted to appropriate values.

Next, functions and effects of the cold storage material particle 10 of the first embodiment will be described.

In a cryogenic refrigerator used for cooling a superconducting device or the like, a cold storage device is filled with a plurality of cold storage material particles. For example, cold is generated by performing heat exchange between the cold storage material particles and helium gas passing through the cold storage device. The cold storage material particle filling the cold storage device is required to have excellent properties such as, for example, high volume specific heat, high mechanical strength, high thermal conductivity, and high heat transfer.

In the cold storage material particle 10 of the first embodiment, a maximum value of volume specific heat at a temperature of 20K or less is 0.3 J/cm³·K or more. Therefore, the cold storage material particle 10 has high volume specific heat at a cryogenic temperature.

In addition, the cold storage material particle 10 of the first embodiment contains an additive metal element that forms a multivalent metal ion in an aqueous solution. The additive metal element has an action of promoting sintering of the particles at the time of sintering in producing the cold storage material particle 10. Therefore, the cold storage material particle 10 has a high degree of sintering and high mechanical strength.

In addition, in order to sufficiently obtain characteristics required for the cold storage material particle 10, such as mechanical strength, thermal conductivity, heat transfer, and volume specific heat, a sufficient sintering temperature and sintering time are required in a sintering process. The sintering promoting action of the additive metal element makes it possible to lower the sintering temperature required for sintering and to reduce the sintering time. Therefore, the manufacturing cost of the cold storage material particle 10 can be reduced, and the inexpensive cold storage material particle 10 can be provided.

In addition, since the cold storage material particle 10 of the first embodiment has a high degree of sintering inside the particle, it has a high thermal conductivity. In addition, since the cold storage material particle 10 of the first embodiment has a high degree of sintering at the outer peripheral portion of the particle, for example, heat transfer characteristics with helium gas in contact with the outer peripheral portion are also improved. Therefore, the cold storage material particle 10 of the first embodiment has high heat transfer.

The atomic concentration of the additive metal element in the cold storage material particle 10 is 0.001 atom% or more and 60 atom% or less. The atomic concentration of the additive metal element in the cold storage material particle 10 is more preferably 0.01 atom% or more and 40 atom% or less.

When the atomic concentration of the additive metal element in the cold storage material particle 10 exceeds the above lower limit value, the degree of sintering increases, and the mechanical strength, thermal conductivity, and heat transfer of the cold storage material particle 10 increase. In addition, when the atomic concentration of the additive metal element in the cold storage material particle 10 is less than the upper limit value, the volume specific heat increases.

The additive metal element in the cold storage material particle 10 is preferably, for example, at least one element selected from the group consisting of calcium (Ca), magnesium (Mg), beryllium (Be), strontium (Sr), barium (Ba), radium (Ra), manganese (Mn), aluminum (Al), iron (Fe), copper (Cu), nickel (Ni), and cobalt (Co). The additive metal element in the cold storage material particle 10 is more preferably at least one element selected from the group consisting of calcium (Ca), magnesium (Mg), beryllium (Be), strontium (Sr), and barium (Ba) .

When the additive metal element is the above element, sintering of the particles at the time of producing the cold storage material particle 10 is further promoted. Therefore, the mechanical strength, thermal conductivity, and heat transfer of the cold storage material particle 10 are increased.

From the viewpoint of increasing the gelation rate of the slurry when the cold storage material particle 10 is produced, the additive metal element is preferably calcium (Ca). From the viewpoint of increasing the mechanical strength, the thermal conductivity, and the heat transfer of the cold storage material particle 10, the additive metal element is preferably aluminum (Al). The additive metal element is preferably calcium (Ca) and aluminum (Al).

The cold storage material particle 10 preferably contains an alkali metal element. Since the cold storage material particle 10 contains the alkali metal element, sintering of the cold storage material particle 10 is promoted in the sintering process. Therefore, sintering of the cold storage material particle 10 is promoted, and the mechanical strength, thermal conductivity, and heat transfer of the cold storage material particle 10 are increased.

In order to sufficiently obtain characteristics required for the cold storage material particle 10 such as the mechanical strength, thermal conductivity, heat transfer, and volume specific heat, a sufficient sintering temperature and sintering time are required in the sintering process. The sintering promoting action of the alkali metal element makes it possible to lower the sintering temperature required for sintering and to reduce the sintering time. Therefore, a manufacturing cost of the cold storage material particle 10 can be reduced, and the inexpensive cold storage material particle 10 can be provided.

The atomic concentration of the alkali metal element in the cold storage material particle 10 is preferably 0.0001 atom% or more and 10 atom% or less, and more preferably 0.001 atom% or more and 5 atom% or less.

In order to include an alkali metal element in the cold storage material particle 10, for example, a sodium alginate aqueous solution or a potassium alginate aqueous solution is used as an alginic acid aqueous solution when the cold storage material particle 10 is produced.

The cold storage material particle 10 preferably includes the first phase containing the cold storage element and the second phase containing the additive metal element and being different from the first phase. For example, as compared with a case where the cold storage material particle 10 is formed of a single phase, a number density of grain boundaries increases due to the presence of different phases.

Furthermore, the cold storage material particle 10 preferably contains the additive metal element and contains the third phase different from the first phase and the second phase. For example, as compared with a case where the cold storage material particle 10 is formed of two different phases, the presence of three different phases suppresses the grain growth of each phase and further increases the number density of grain boundaries. Here, the number density of grain boundaries means the number of grain boundaries per unit volume.

Furthermore, the cold storage material particle 10 preferably contains the additive metal element and contains a fourth phase different from the first phase, the second phase, and the third phase. For example, as compared with a case where the cold storage material particle 10 is formed of three different phases, the presence of four different phases further suppresses the grain growth of each phase and dramatically increases the number density of grain boundaries.

As the number density of grain boundaries increases, the strength of the cold storage material particle 10 against plastic deformation increases. Therefore, inclusion of the second phase different from the first phase increases the mechanical strength of the cold storage material particle 10. The inclusion of the third phase in addition to the second phase further increases the mechanical strength of the cold storage material particle 10. By including the third phase and the fourth phase in addition to the second phase, the mechanical strength of the cold storage material particle 10 is further enhanced.

The cold storage material particle 10 may have a form in which the additive metal element is uniformly dispersed in the cold storage material particle 10, and the cold storage material particle is composed of a single phase. Here, uniform dispersion means that the additive metal element is uniformly dispersed in the cold storage material particle 10 at an atomic level, and the atomic concentration of the additive metal element is substantially constant over the entire cold storage material particle 10.

When the cold storage material particle 10 includes the first phase and the second phase, in the cross section of the cold storage material particle 10, the proportion of the area occupied by the second phase is preferably 0.01% or more and 75% or less, and more preferably 0.01% or more and 50% or less.

In the cross section of the cold storage material particle 10, when the proportion of the area occupied by the second phase exceeds the lower limit value, the number density of the grain boundaries increases, and the mechanical strength of the cold storage material particle 10 increases. In addition, the degree of sintering of the cold storage material particle 10 increases, and the mechanical strength, the thermal conductivity, and the heat transfer increase. In addition, when the proportion of the area occupied by the second phase in the cold storage material particle 10 is less than the upper limit value, the proportion occupied by the cold storage substance in the cold storage material particle 10 increases, and the volume specific heat increases.

In the cross section of the cold storage material particle 10, the area per second phase is preferably 0.001 µm² or more and 6000 µm² or less. In the cross section of the cold storage material particle 10, when the area of the second phase exceeds the above lower limit value, the number density of grain boundaries decreases, and the thermal conductivity of the cold storage material particle 10 is improved. In addition, when the area per second phase in the cold storage material particle 10 is less than the upper limit value, the number density of grain boundaries in the cold storage material particle 10 increases, and the mechanical strength of the cold storage material particle 10 increases.

In the cross section of the cold storage material particle 10, the particle diameter per second phase is preferably 0.1 um or more and 100 um or less. In the cross section of the cold storage material particle 10, when the particle diameter of the second phase exceeds the above lower limit value, the number density of grain boundaries decreases, and the thermal conductivity of the cold storage material particle 10 is improved. In addition, when the particle diameter per second phase in the cold storage material particle 10 is less than the upper limit value, the number density of grain boundaries in the cold storage material particle 10 increases, and the mechanical strength of the cold storage material particle 10 increases.

In the cross section of the cold storage material particle 10, preferably 20% or more and 90% or less of the second phase is in contact with the third phase, and preferably 40% or more and 75% or less of the second phase is in contact with the third phase. In other words, the proportion of the second phase in contact with the third phase in the second phase is preferably 20% or more and 90% or less, and more preferably 40% or more and 75% or less.

When the second phase and the third phase are in contact with each other, grain growth of the second phase and the third phase during sintering is suppressed. Therefore, it is possible to increase the sintering temperature or increase the sintering time while finely maintaining the second phase and the third phase.

Therefore, the proportion of voids present in the cold storage material particles can be reduced. That is, the porosity present in the cold storage material particles can be reduced while keeping the number density of the grain boundary phase high. Therefore, when the proportion of the second phase in contact with the third phase exceeds the lower limit, the mechanical strength of the cold storage material particle is improved.

It is considered that thermal expansion coefficients of the second phase and the third phase are different. Therefore, when cooling is performed from room temperature to low temperature during operation of the refrigerator, stress is generated between the second phase and the third phase due to a difference in thermal shrinkage. Similarly, the thermal expansion coefficients of the second phase and the fourth phase are considered to be different. Therefore, when cooling is performed from room temperature to low temperature during operation of the refrigerator, stress is generated between the second phase and the fourth phase due to a difference in thermal shrinkage.

When the second phase is in contact with the third phase and the fourth phase at the same time, the second phase is simultaneously subjected to stress due to a difference in thermal shrinkage between the third phase and the fourth phase, and the respective stresses become large in combination. Therefore, when the proportion of the second phase in contact with the third phase is less than the upper limit value, the proportion of the second phase in contact with the third phase and the fourth phase at the same time can be reduced. As a result, when the cold storage material particle 10 is cooled from room temperature to a low temperature during operation of the refrigerator, it is possible to suppress generation of cracks in the particle due to a difference in thermal shrinkage.

In the cross section of the cold storage material particle 10, the proportion of the second phase in contact with the third phase in the second phase is preferably higher than the proportion of the second phase in contact with the fourth phase. By increasing the proportion of the second phase in contact with the third phase, the mechanical strength of the cold storage material particle 10 is improved.

The particle diameter of the cold storage material particle 10 is preferably 50 um or more and 3 mm or less, more preferably 1 mm or less, and still more preferably 500 um or less. When the particle diameter of the cold storage material particle 10 exceeds the above lower limit value, packing density of the cold storage material particle in the cold storage device is lowered, a pressure loss of the working medium such as helium is reduced, and refrigeration performance of the refrigerator is improved. Meanwhile, when the particle diameter of the cold storage material particle 10 is less than the upper limit value, the distance from the surface of the cold storage material particle to the central portion of the particle is shortened, heat transfer between the working medium and the cold storage material particle is easily performed to the central portion of the cold storage material, and the refrigeration performance of the refrigerator is improved.

The aspect ratio of the cold storage material particle 10 is preferably 5 or less, and more preferably 2 or less. When the aspect ratio of the cold storage material particle 10 is less than the above upper limit value, the gap becomes uniform when the cold storage material fills the cold storage device, and the refrigeration performance of the refrigerator is improved.

By controlling the area of each phase present in the particle, the particle diameter, and the contact ratio of each phase by the method for producing a cold storage material particle of the first embodiment, it is possible to reduce variations in mechanical strength and thermal conductivity of the cold storage material particle.

As described above, according to the first embodiment, it is possible to realize the cold storage material particle having excellent characteristics such as high volume specific heat, high mechanical strength, high thermal conductivity, and high heat transfer.

### (Second Embodiment)

A cold storage material particle of a second embodiment is different from the cold storage material particle of the first embodiment in that the cold storage material particle does not include the fourth phase. Hereinafter, description of contents overlapping with the first embodiment will be partially omitted.

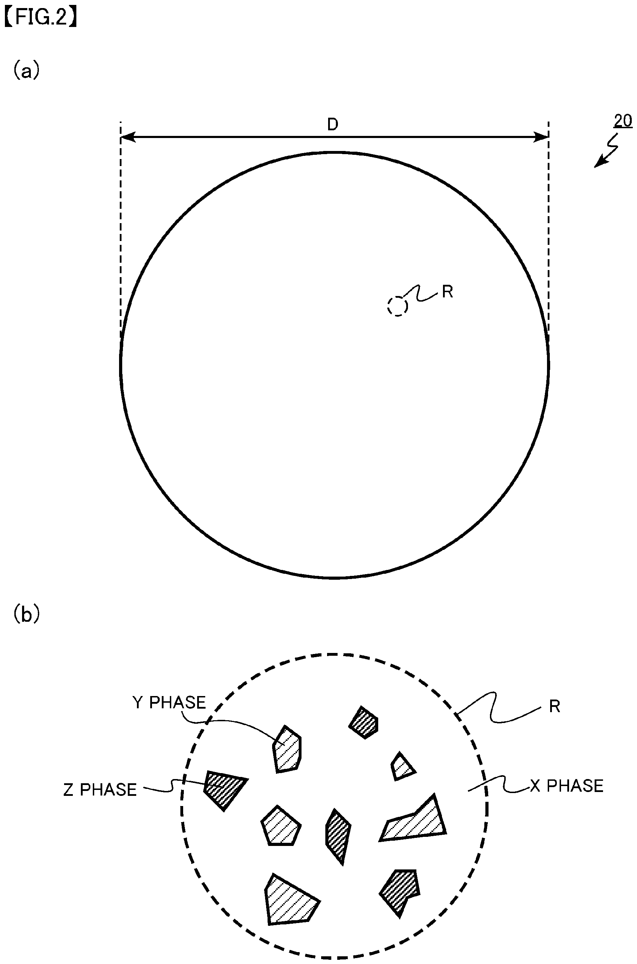

FIG. 2 is an explanatory view of the cold storage material particle according to the second embodiment. FIG. 2(a) is a schematic cross-sectional view of the cold storage material particle. FIG. 2(b) is a schematic cross-sectional view in which a part of the cold storage material particle is enlarged. FIG. 2(b) is a schematic cross-sectional view of a region R in FIG. 2(a), for example.

As illustrated in FIG. 2(b), the cold storage material particle 20 of the second embodiment includes three phases of an X phase, a Y phase, and a Z phase. The X phase, the Y phase, and the Z phase are different phases. The X phase, the Y phase, and the Z phase are examples of a first phase, a second phase, and a third phase, respectively.

The X phase, the Y phase, and the Z phase are, for example, crystal phases. The X phase, the Y phase, and the Z phase may be, for example, an amorphous phase.

The X phase is a cold storage substance. The X phase contains a cold storage element. The X phase is, for example, gadolinium oxysulfide. The X phase is, for example, Gd₂O₂S.

When the X phase is gadolinium oxysulfide, the cold storage element is gadolinium (Gd). The X phase contains gadolinium (Gd), sulfur (S), and oxygen (O).

The Y phase contains an additive metal element. An atomic concentration of the additive metal element in the Y phase is larger than an atomic concentration of the additive metal element in the X phase.

The Y phase is, for example, an oxide containing aluminum (Al). The Y phase is, for example, aluminum oxide. The Y phase is, for example, Al₂O₃.

When the Y phase is an oxide containing aluminum oxide, the additive metal element is aluminum (Al).

The Z phase contains a cold storage element and an additive metal element. The Z phase is, for example, an oxide containing gadolinium (Gd) and aluminum (Al). The Z phase is, for example, a compound containing gadolinium (Gd), aluminum (Al), and oxygen (O). The Z phase is, for example, GdAlO₃.

An atomic concentration of the additive metal element in the second phase is preferably larger than an atomic concentration of the additive metal element in the first phase. For example, an atomic concentration of the additive metal element in the Y phase is preferably larger than an atomic concentration of the additive metal element in the X phase.

It is preferable that 20% or more and 90% or less of the second phase is in contact with the third phase. For example, it is preferable that 20% or more and 90% or less of the Y phase is in contact with the Z phase.

In the cross section of the cold storage material particle, a proportion of an area occupied by the second phase is preferably 0.001% or more and 75% or less. For example, in the cross section of the cold storage material particle 20, the proportion of the area occupied by the Y phase is preferably 0.001% or more and 75% or less.

In the cross section of the cold storage material particle, the area per second phase is preferably 0.001 µm² or more and 6000 µm² or less. For example, in the cross section of the cold storage material particle 20, the area per Y phase is preferably 0.001 µm² or more and 6000 µm² or less.

In the cross section of the cold storage material particle, the particle diameter per second phase is preferably 0.1 um or more and 100 um or less. For example, in the cross section of the cold storage material particle 20, the particle diameter per Y phase is preferably 0.1 um or more and 100 um or less.

The cold storage material particle 20 of the second embodiment can be manufactured by the same producing method as the producing method of the first embodiment.

As described above, according to the second embodiment, similarly to the first embodiment, the cold storage material particle having excellent characteristics of high volume specific heat, high mechanical strength, high thermal conductivity, and high heat transfer can be realized.

### (Third Embodiment)

A cold storage material particle of a third embodiment is different from that of the first embodiment in that the cold storage material particle has a first region and a second region that is closer to an outer edge of the cold storage material particle than the first region and has a higher atomic concentration of the second element than that of the first region. Hereinafter, description of contents overlapping with the first embodiment will be partially omitted.

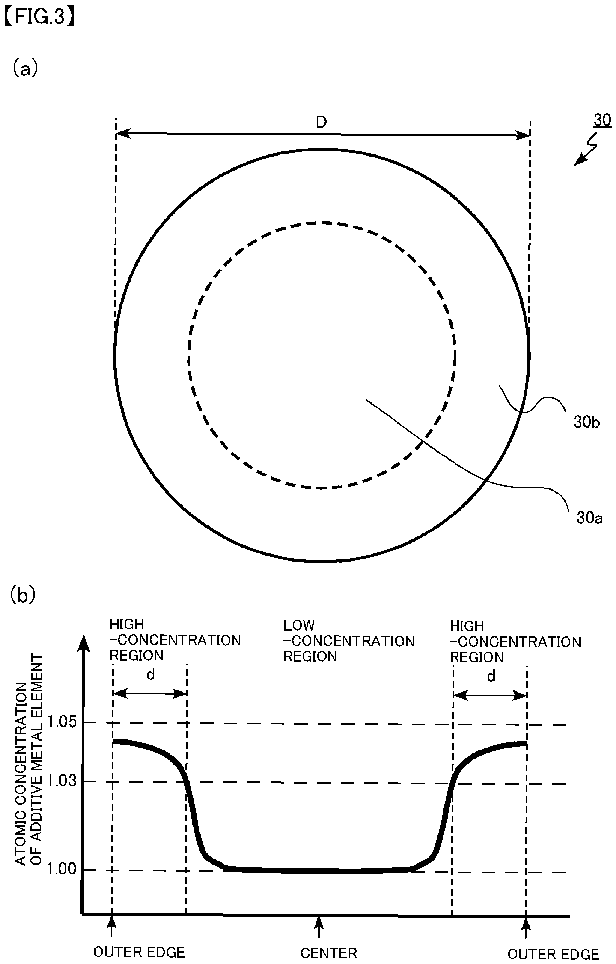

FIG. 3 is an explanatory view of the cold storage material particle according to the third embodiment. FIG. 3(a) is a schematic cross-sectional view of the cold storage material particle. FIG. 3(b) is a diagram illustrating an atomic concentration distribution of an additive metal element in the cold storage material particle.

A cold storage material particle 30 has a low-concentration region 30a (first region) and a high-concentration region 30b (second region). The atomic concentration of the additive metal element in the high-concentration region 30b is higher than the atomic concentration of the additive metal element in the low-concentration region 30a.

The high-concentration region 30b is closer to an outer edge of the cold storage material particle 30 than the low-concentration region 30a. The high-concentration region 30b surrounds the low-concentration region 30a. The low-concentration region 30a is, for example, a region including a center of the cold storage material particle 30, and the high-concentration region 30b is a region of an outer periphery of the low-concentration region 30a.

The low-concentration region 30a and the high-concentration region 30b contain a cold storage element. At least in the high-concentration region 30b, the cold storage element and the additive metal element are mixed. It is also possible to adopt a structure in which the low-concentration region 30a does not contain an additive metal element.

The atomic concentration of the additive metal element in the high-concentration region 30b is, for example, 0.1 atom% or more and 2.0 atom% or less.

The atomic concentration of the additive metal element in the high-concentration region 30b is, for example, 1.03 times or more and 10 times or less the atomic concentration of the additive metal element in the low-concentration region 30a. A distance (d in FIG. 3(b)) from the outer edge of the cold storage material particle 30 in the high-concentration region 30b where the atomic concentration of the additive metal element in the high-concentration region 30b is 1.03 times or more the atomic concentration of the additive metal element in the low-concentration region 30a is, for example, 1/20 or more of the particle diameter D of the cold storage material particle 30.

The distance (d in FIG. 3(b)) from the outer edge of the cold storage material particle 30 to a position where the atomic concentration of the additive metal element is 1.03 times or more the atomic concentration of the additive metal element in the low-concentration region 30a is, for example, 10 um or more.

The atomic concentration of the additive metal element monotonously decreases, for example, from the outer edge toward the center of the cold storage material particle 30.

The detection of the additive metal element contained in the cold storage material particle 30 and the measurement of the atomic concentration of the additive metal element can be performed by, for example, WDX. For example, it is possible to measure the additive metal element concentrations at a plurality of places from the outer edge of the cold storage material particle 30 toward the center by WDX, determine whether or not the high-concentration region 30b close to the outer edge of the cold storage material particle 30 exists, determine the atomic concentration of the additive metal element in the high-concentration region 30b, and calculate the proportion of the atomic concentration of the additive metal element in the high-concentration region 30b to the atomic concentration of the additive metal element in the low-concentration region 30a, and the distance (d in FIG. 3(b)) from the outer edge of the cold storage material particle 30 to the position where the atomic concentration of the additive metal element is 1.03 times or more the atomic concentration of the additive metal element in the low-concentration region 30a. In addition, for example, the atomic concentration of the additive metal element in the cold storage material particle 30 is mapped by WDX, and it is possible to identify whether or not the high-concentration region 30b surrounds the low-concentration region 30a.

The cold storage material particle 30 of the third embodiment can be manufactured by the same producing method as the producing method of the first embodiment.

When the cold storage material particle 30 of the third embodiment is manufactured, for example, the atomic concentration distribution of the additive metal element in the particle is controlled by controlling the gelation time. That is, by controlling the gelation time, a distribution in which the atomic concentration of the additive metal element in the central region of the particle is low and the atomic concentration of the additive metal element in the outer peripheral region of the particle is high is formed.

From the viewpoint of forming the distribution, the gelation time is preferably as short as possible as long as the shape of the particles does not collapse in the subsequent step. The gelation time is preferably 1 hour or less, and more preferably 30 minutes or less.

Next, functions and effects of the cold storage material particle 30 of the third embodiment will be described.

The cold storage material particle 30 of the third embodiment includes the high-concentration region 30b in which the atomic concentration of the additive metal element is high in the outer peripheral region of the particle. The additive metal element has an action of promoting sintering of the particles at the time of sintering in producing the cold storage material particle 30. Therefore, the high-concentration region 30b has a high degree of sintering and high mechanical strength. Therefore, the cold storage material particle 30 has high mechanical strength.

In addition, since the high-concentration region 30b has a high degree of sintering, the thermal conductivity and the heat transfer of the high-concentration region 30b are increased. Therefore, the cold storage material particle 30 has high thermal conductivity and high heat transfer.

When the atomic concentration of the additive metal element increases, the degree of sintering of the particles increases, but there is a problem that a volume ratio of the cold storage substance decreases as the volume ratio of the additive metal element increases. In addition, the additive metal element forms a compound having low volume specific heat by reaction with the cold storage substance. Therefore, when the atomic concentration of the additive metal element increases, the cold storage substance may be changed to a compound having low volume specific heat, and the volume ratio of the cold storage substance may decrease.

The cold storage material particle 30 of the third embodiment includes a low-concentration region 30a in which the atomic concentration of the additive metal element is low in the central region of the particle. Therefore, in the central region of the particle, a decrease in the volume ratio of the cold storage substance due to the additive metal element is suppressed. Therefore, the cold storage material particle 30 has high volume specific heat.

The cold storage material particle 30 of the third embodiment includes the high-concentration region 30b having a high atomic concentration of the additive metal element in the outer peripheral region of the particle, so that the mechanical strength, the thermal conductivity, and the heat transfer are improved. Meanwhile, the volume specific heat is improved by providing the low-concentration region 30a in the central region of the particle. The cold storage material particle 30 of the third embodiment has high volume specific heat, high mechanical strength, high thermal conductivity, and high heat transfer by optimizing the concentration distribution of the additive element in the particle.

The atomic concentration of the additive metal element in the high-concentration region 30b is preferably 0.1 atom% or more, and more preferably 0.2 atom% or more. It is possible to increase the mechanical strength, thermal conductivity, and heat transfer of the cold storage material particle 30.

The atomic concentration of the additive metal element in the high-concentration region 30b is preferably 1.03 times or more, more preferably 1.05 times or more, still more preferably 1.1 times or more, and most preferably 1.2 times or more the atomic concentration of the additive metal element in the low-concentration region 30a. It is possible to realize the high mechanical strength, high thermal conductivity, high heat transfer, and high volume specific heat of the cold storage material particle 30.

The distance (d in FIG. 3(b)) from the outer edge of the cold storage material particle 30 to the position where the atomic concentration of the additive metal element is 1.03 times or more the added-metal concentration in the low-concentration region 30a is preferably 1/20 or more, more preferably 1/10 or more of the particle diameter D (D in FIG. 3(a)) of the cold storage material particle 30. It is possible to increase the mechanical strength, thermal conductivity, and heat transfer of the cold storage material particle 30.

The distance (d in FIG. 3(b)) from the outer edge of the cold storage material particle 30 to the position where the atomic concentration of the additive metal element is 1.03 times or more the added-metal concentration in the low-concentration region 30a is preferably 10 um or more, and more preferably 20 um or more. It is possible to increase the mechanical strength, thermal conductivity, and heat transfer of the cold storage material particle 30.

From the viewpoint of achieving the high mechanical strength, high thermal conductivity, high heat transfer, and high volume specific heat of the cold storage material particle 30, the added-metal concentration preferably monotonously decreases from the outer edge toward the center of the cold storage material particle 30.

As described above, according to the third embodiment, it is possible to realize cold storage material particles having excellent characteristics such as the high volume specific heat, high mechanical strength, high thermal conductivity, and high heat transfer.

### (Fourth Embodiment)

A refrigerator according to a fourth embodiment is a refrigerator including a cold storage device filled with the plurality of cold storage material particles according to the first embodiment. Hereinafter, description of contents overlapping with the first embodiment will be partially omitted.

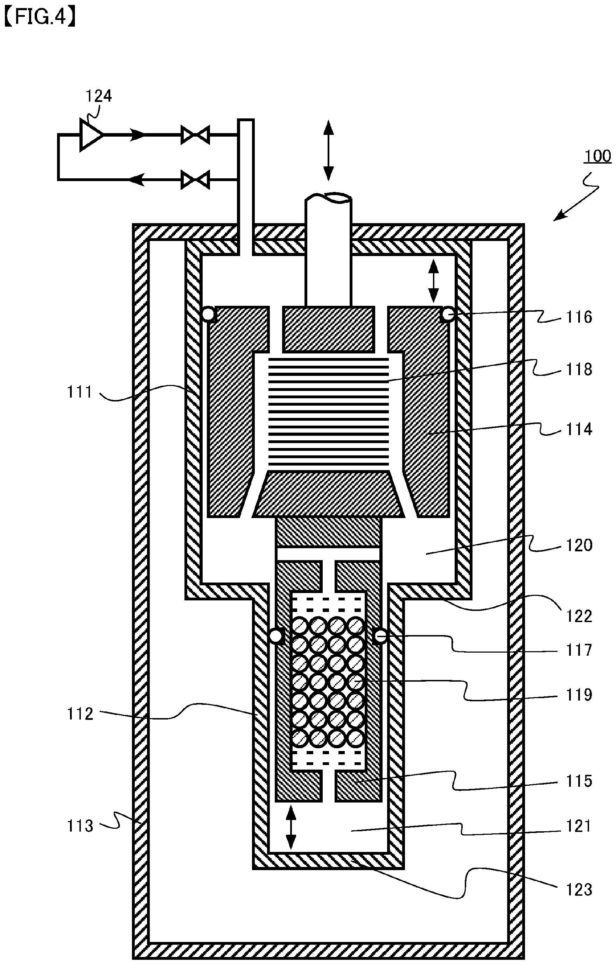

FIG. 4 is a schematic cross-sectional view illustrating a configuration of a main part of a GM refrigerator which is an example of the refrigerator according to the fourth embodiment. The refrigerator of the fourth embodiment is a two-stage cold storage type cryogenic refrigerator 100 used for cooling a superconducting device or the like. The cold storage device filled with the plurality of cold storage material particles of the first embodiment may be a Stirling type refrigerator or a pulse tube type refrigerator in addition to the GM refrigerator described above.

The cold storage type cryogenic refrigerator 100 includes a first cylinder 111, a second cylinder 112, a vacuum container 113, a first cold storage device 114, a second cold storage device 115, a first seal ring 116, a second seal ring 117, a first cold storage material 118, a second cold storage material 119, a first expansion chamber 120, a second expansion chamber 121, a first cooling stage 122, a second cooling stage 123, and a compressor 124.

The cold storage type cryogenic refrigerator 100 includes the vacuum container 113 in which the large-diameter first cylinder 111 and the small-diameter second cylinder 112 coaxially connected to the first cylinder 111 are installed. The first cold storage device 114 is disposed in the first cylinder 111 so as to be reciprocable. In the second cylinder 112, the second cold storage device 115, which is an example of the cold storage device of the fourth embodiment, is disposed so as to be reciprocable.

A first seal ring 116 is disposed between the first cylinder 111 and the first cold storage device 114. The second seal ring 117 is disposed between the second cylinder 112 and the second cold storage device 115.

The first cold storage device 114 accommodates the first cold storage material 118 such as a Cu mesh. The second cold storage device 115 is filled with a plurality of the cold storage material particles 10 of the first embodiment as the second cold storage material 119.

The second cold storage device 115 may be divided by a metal mesh material and include a plurality of cold storage material filling layers. When the second cold storage device 115 is divided into a plurality of filling layers, at least one filling layer is filled with a cold storage material particle group including a plurality of cold storage material particles of the first embodiment, and is combined with, for example, at least one cold storage material particle group selected from a lead cold storage material particle group, a bismuth cold storage material particle group, a tin cold storage material particle group, a holmium copper cold storage material particle group, an erbium nickel cold storage material particle group, an erbium cobalt cold storage material particle group, and a gadolinium aluminum oxide cold storage material particle group.

In the combination of the cold storage materials, the one having a higher peak temperature of specific heat is defined as a first cold storage material particle group, the one having a lower peak temperature of specific heat is defined as a second cold storage material particle group, and the combination is performed so that the peak temperature of specific heat is sequentially lowered.

In the case of the two-layer type, a combination using a holmium copper cold storage material particle group for the first cold storage material particle group and the cold storage material particle group according to the first embodiment for the second cold storage material particle group can be mentioned. Further, in the case of the three-layer type, a combination in which at least one kind of cold storage material particle group selected from a lead cold storage material particle group, a bismuth cold storage material particle group, and a tin cold storage material particle group is used as the first cold storage material particle group, a holmium copper cold storage material particle group is used as the second cold storage material particle group, and the cold storage material particle group according to the first embodiment is used as the third cold storage material particle group, and the like can be mentioned.

The holmium copper cold storage material particle is preferably, for example, HoCu₂ or HoCu. The erbium-nickel cold storage material particle is preferably, for example, ErNi or Er₃Ni.

The first cold storage device 114 and the second cold storage device 115 each have a working medium passage provided in a gap between the first cold storage material 118 and the second cold storage material 119. The working medium is helium gas.

The first expansion chamber 120 is provided between the first cold storage device 114 and the second cold storage device 115. The second expansion chamber 121 is provided between the second cold storage device 115 and a distal end wall of the second cylinder 112. The first cooling stage 122 is provided at the bottom of the first expansion chamber 120. The second cooling stage 123 having a temperature lower than that of the first cooling stage 122 is formed at the bottom of the second expansion chamber 121.

A high-pressure working medium is supplied from the compressor 124 to the above-described two-stage cold storage type cryogenic refrigerator 100. The supplied working medium passes between the first cold storage materials 118 housed in the first cold storage device 114 and reaches the first expansion chamber 120. Then, the refrigerant passes between the second cold storage materials 119 housed in the second cold storage device 115 and reaches the second expansion chamber 121.

At this time, the working medium is cooled by supplying thermal energy to the first cold storage material 118 and the second cold storage material 119. The working medium passing between the first cold storage material 118 and the second cold storage material 119 expands in the first expansion chamber 120 and the second expansion chamber 121 to generate cold. Then, the first cooling stage 122 and the second cooling stage 123 are cooled.

The expanded working medium flows in the opposite direction between the first cold storage material 118 and the second cold storage material 119. The working medium is discharged after receiving thermal energy from the first cold storage material 118 and the second cold storage material 119. The cold storage type cryogenic refrigerator 100 is configured such that the thermal efficiency of the working medium cycle is improved as the recuperating effect is improved in such a process, and a lower temperature is realized.

By using the refrigerator of the fourth embodiment for a magnetically levitated train, a helium re-condensing device, and the like, it is possible to improve the long-term reliability of the magnetically levitated train and the helium re-condensing device.

The cold storage material particle of the second embodiment or the third embodiment can also be applied as the second cold storage material 119.

As described above, according to the fourth embodiment, it is possible to realize the refrigerator having excellent characteristics by using cold storage material particles having excellent characteristics.

### (Fifth Embodiment)

A cryopump according to a fifth embodiment includes the refrigerator according to the fourth embodiment. Hereinafter, description of contents overlapping with the fourth embodiment will be partially omitted.

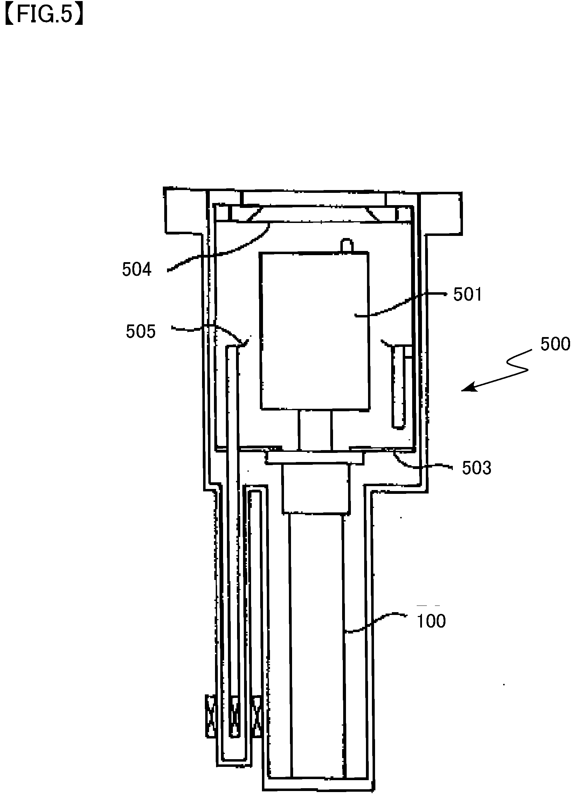

FIG. 5 is a cross-sectional view illustrating a schematic configuration of the cryopump according to the fifth embodiment. The cryopump according to the fifth embodiment is a cryopump 500 including the cold storage type cryogenic refrigerator 100 according to the fourth embodiment.

The cryopump 500 includes a cryopanel 501 that condenses or adsorbs gas molecules, a cold storage type cryogenic refrigerator 100 that cools the cryopanel 501 to a predetermined cryogenic temperature, a shield 503 provided between the cryopanel 501 and the cold storage type cryogenic refrigerator 100, a baffle 504 provided at an intake port, and a ring 505 that changes an exhaust speed of argon, nitrogen, hydrogen, or the like.

According to the fifth embodiment, a cryopump having excellent characteristics can be realized by using a refrigerator having excellent characteristics. In addition, by using the cryopump according to the fifth embodiment in a semiconductor manufacturing apparatus or the like, long-term reliability of the semiconductor manufacturing apparatus can be improved, and the number of times of maintenance of the semiconductor manufacturing apparatus can be reduced. As a result, this contributes to improvement in quality of a semiconductor to be manufactured and reduction in manufacturing cost.

### (Sixth Embodiment)

A superconducting magnet of the sixth embodiment includes the refrigerator of the fourth embodiment. Hereinafter, description of contents overlapping with the fourth embodiment will be partially omitted.

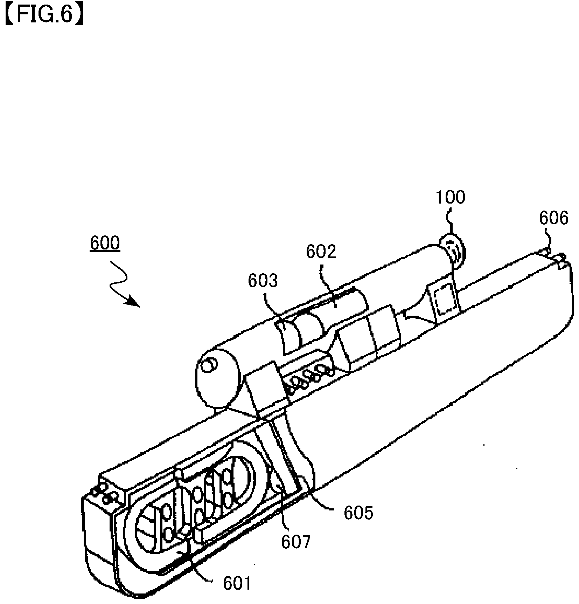

FIG. 6 is a perspective view illustrating a schematic configuration of the superconducting magnet according to the sixth embodiment. The superconducting magnet of the sixth embodiment is a superconducting magnet 600 for a magnetically levitated train including the cold storage type cryogenic refrigerator 100 of the fourth embodiment.

The superconducting magnet 600 for a magnetically levitated train includes a superconducting coil 601, a liquid helium tank 602 for cooling the superconducting coil 601, a liquid nitrogen tank 603 for preventing volatilization of liquid helium, a laminated heat insulating material 605, a power lead 606, a permanent current switch 607, and a cold storage type cryogenic refrigerator 100.

According to the sixth embodiment, a superconducting magnet having excellent characteristics can be realized by using a refrigerator having excellent characteristics.

### (Seventh Embodiment)

A nuclear magnetic resonance imaging apparatus of a seventh embodiment includes the refrigerator of the fourth embodiment. Hereinafter, description of contents overlapping with the fourth embodiment will be partially omitted.

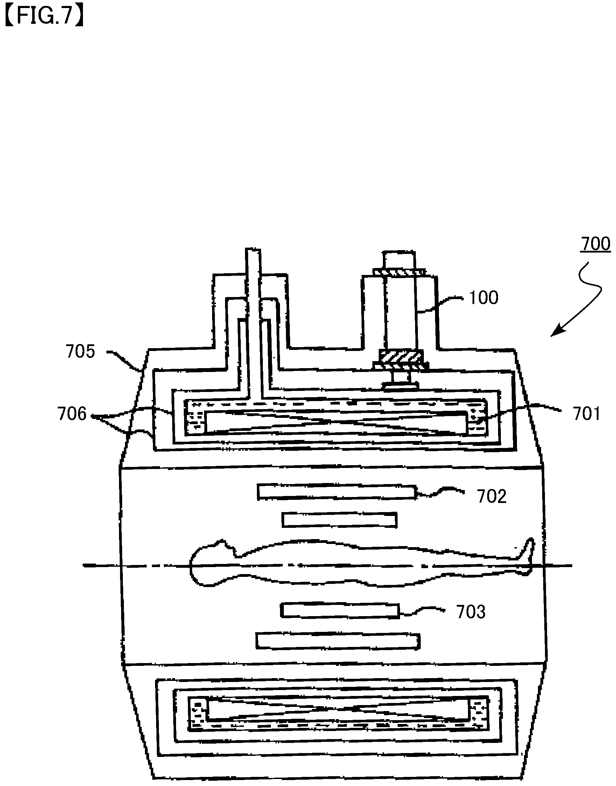

FIG. 7 is a cross-sectional view illustrating a schematic configuration of the nuclear magnetic resonance imaging apparatus of the seventh embodiment. The nuclear magnetic resonance imaging (MRI) apparatus of the seventh embodiment is a nuclear magnetic resonance imaging apparatus 700 including the cold storage type cryogenic refrigerator 100 of the fourth embodiment.

The nuclear magnetic resonance imaging apparatus 700 includes a superconducting static magnetic field coil 701 that applies a spatially uniform and temporally stable static magnetic field to a human body, a correction coil (not illustrated) that corrects nonuniformity of a generated magnetic field, a gradient magnetic field coil 702 that gives a magnetic field gradient to a measurement region, a radio wave transmission/reception probe 703, a cryostat 705, and a radiation adiabatic shield 706. The cold storage type cryogenic refrigerator 100 is used for cooling the superconducting static magnetic field coil 701.

According to the seventh embodiment, a nuclear magnetic resonance imaging apparatus having excellent characteristics can be realized by using a refrigerator having excellent characteristics.

### (Eighth Embodiment)

The nuclear magnetic resonance apparatus of an eighth embodiment includes the refrigerator of the fourth embodiment. Hereinafter, description of contents overlapping with the fourth embodiment will be partially omitted.

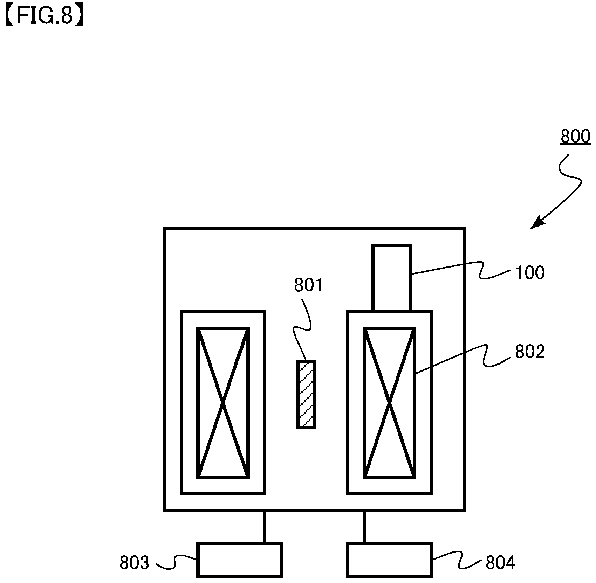

FIG. 8 is a cross-sectional view illustrating a schematic configuration of the nuclear magnetic resonance apparatus of the eighth embodiment. The nuclear magnetic resonance (NMR) apparatus of the eighth embodiment is a nuclear magnetic resonance apparatus 800 including the cold storage type cryogenic refrigerator 100 of the fourth embodiment.

The nuclear magnetic resonance apparatus 800 includes a superconducting static magnetic field coil 802 that applies a magnetic field to a sample such as an organic substance placed in a sample tube 801, a high frequency oscillator 803 that applies a radio wave to the sample tube 801 in the magnetic field, and an amplifier 804 that amplifies an induced current generated in a coil (not illustrated) around the sample tube 801. In addition, the cold storage type cryogenic refrigerator 100 that cools the superconducting static magnetic field coil 802 is provided.

According to the eighth embodiment, a nuclear magnetic resonance apparatus having excellent characteristics can be realized by using a refrigerator having excellent characteristics.

### (Ninth Embodiment)

A magnetic field application type single crystal pulling apparatus of a ninth embodiment includes the refrigerator of the fourth embodiment. Hereinafter, description of contents overlapping with the fourth embodiment will be partially omitted.

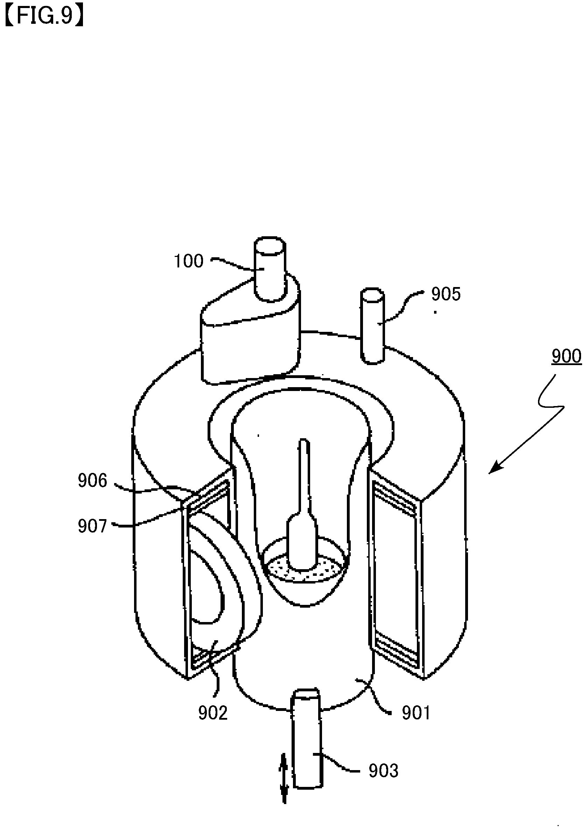

FIG. 9 is a perspective view illustrating a schematic configuration of the magnetic field application type single crystal pulling apparatus according to the ninth embodiment; The magnetic field application type single crystal pulling apparatus of the ninth embodiment is a magnetic field application type single crystal pulling apparatus 900 including the cold storage type cryogenic refrigerator 100 of the ninth embodiment.

The magnetic field application type single crystal pulling apparatus 900 includes a single crystal pulling unit 901 having a raw material melting crucible, a heater, a single crystal pulling mechanism, and the like, a superconducting coil 902 that applies a static magnetic field to the raw material melt, a lifting mechanism 903 of the single crystal pulling unit 901, a current lead 905, a heat shield plate 906, and a helium container 907. The cold storage type cryogenic refrigerator 100 is used for cooling the superconducting coil 902.

According to the ninth embodiment, the magnetic field application type single crystal pulling apparatus having excellent characteristics can be realized by using the refrigerator having excellent characteristics.

The refrigerator of the fourth embodiment can improve the long-term reliability by being used in a magnetically levitated train, a helium re-condensing device, and the like.

### EXAMPLES

Hereinafter, examples, comparative examples, and evaluation results thereof of the cold storage material particles of the first to third embodiments will be described.

Configurations and evaluation results of cold storage material particles of Examples 1 to 77 and Comparative Examples 1 to 7 are illustrated in Tables 1 to 7 .

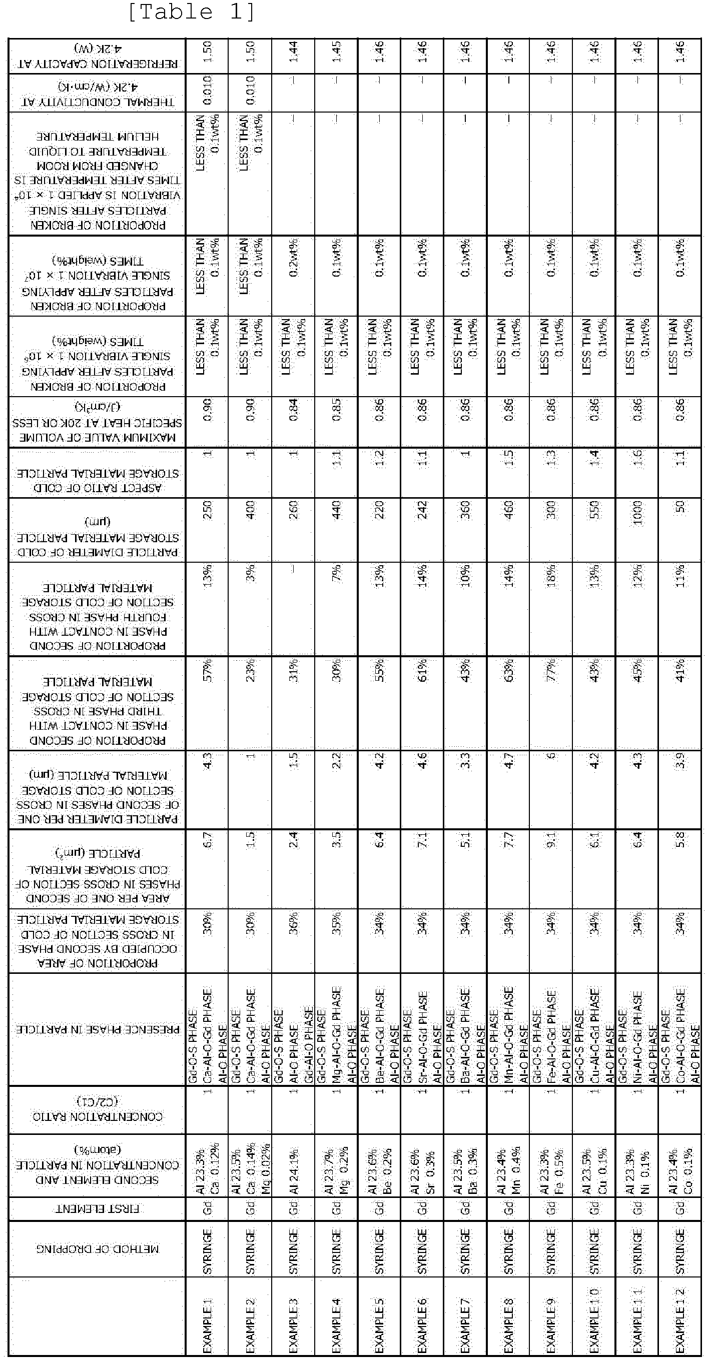

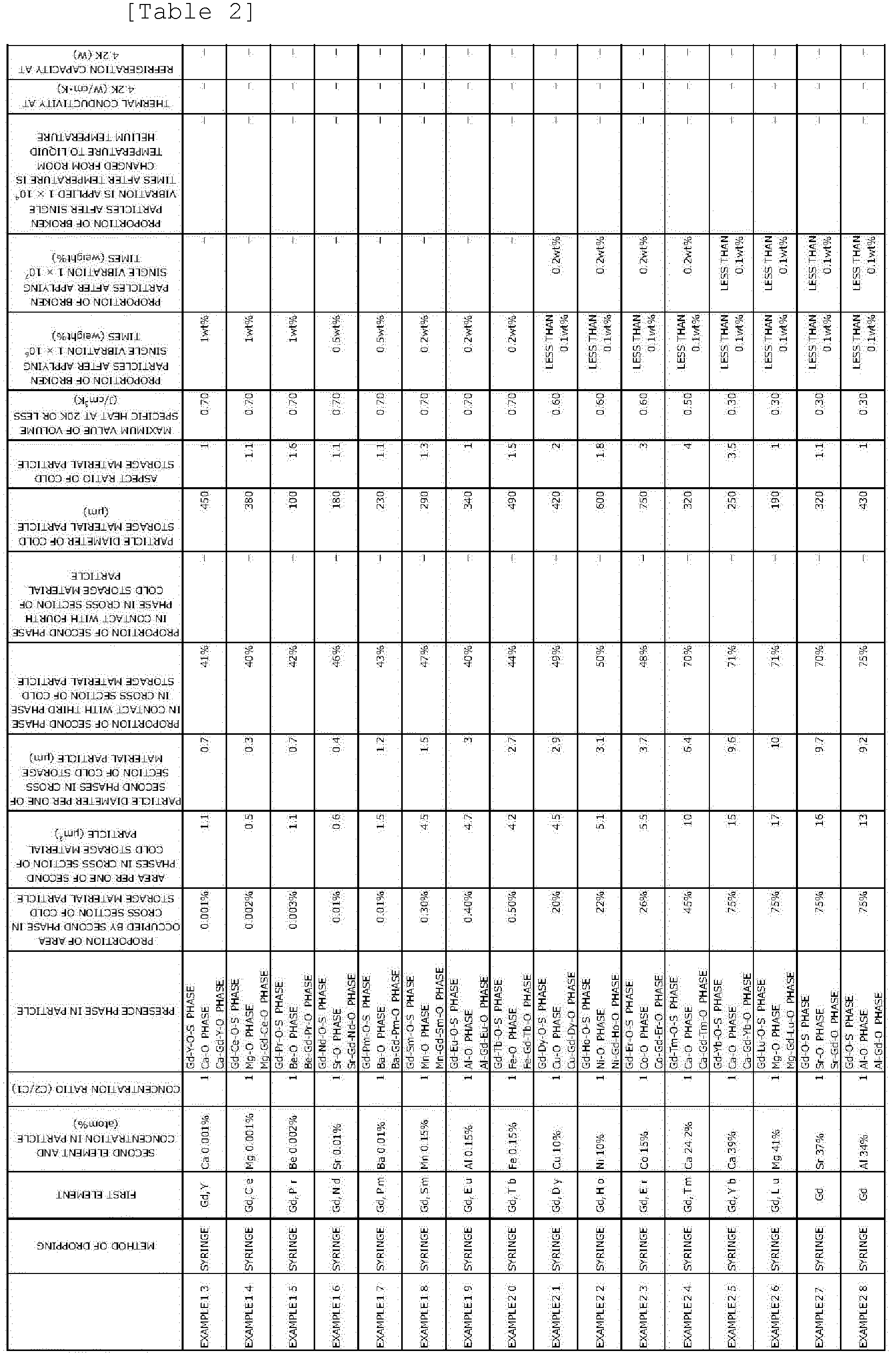

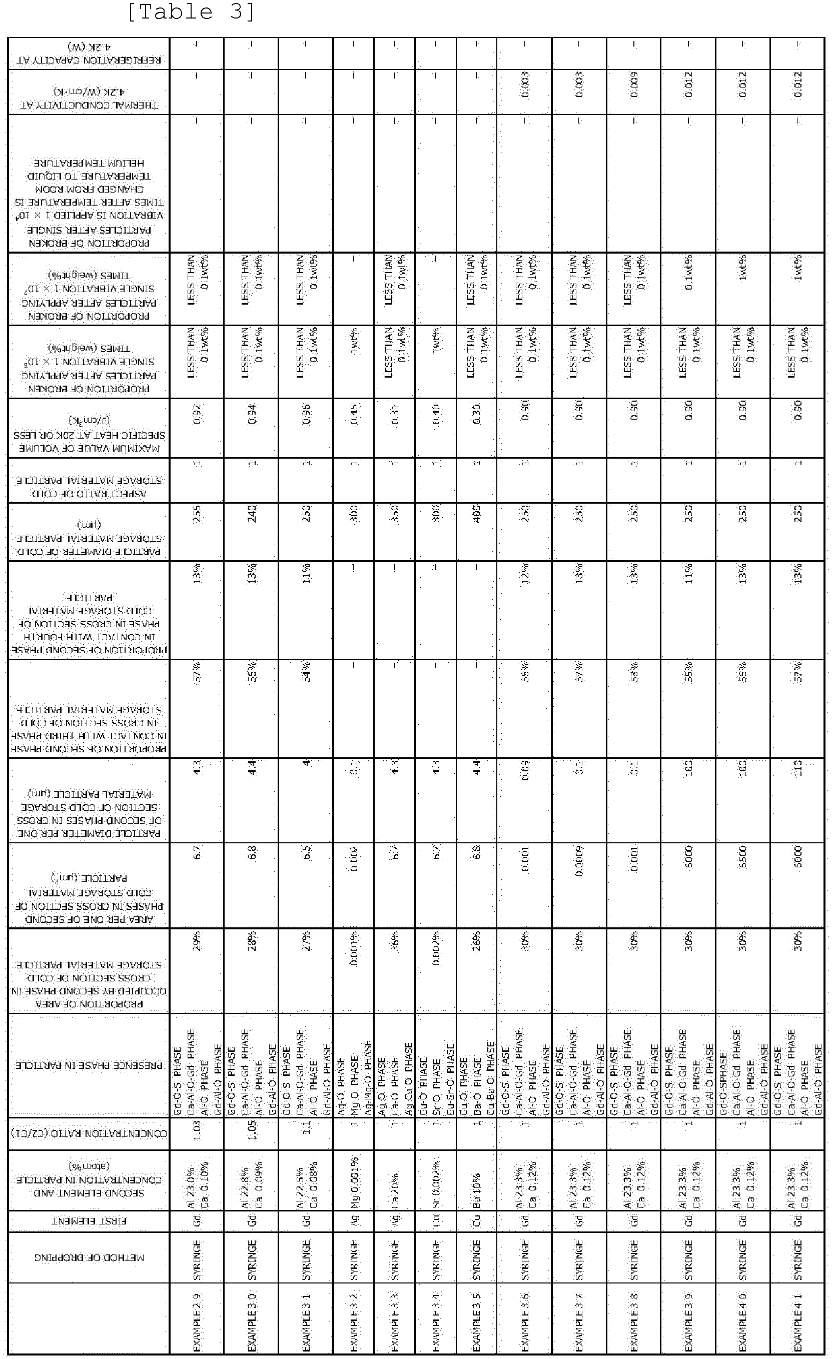

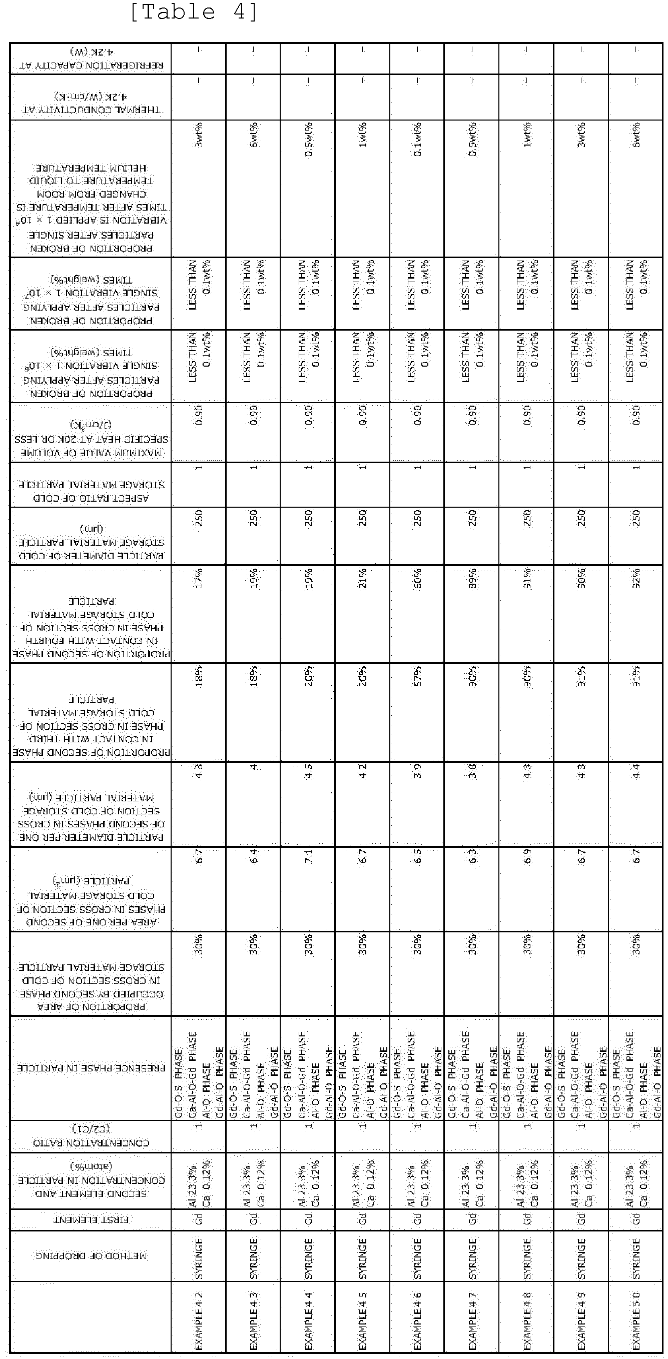

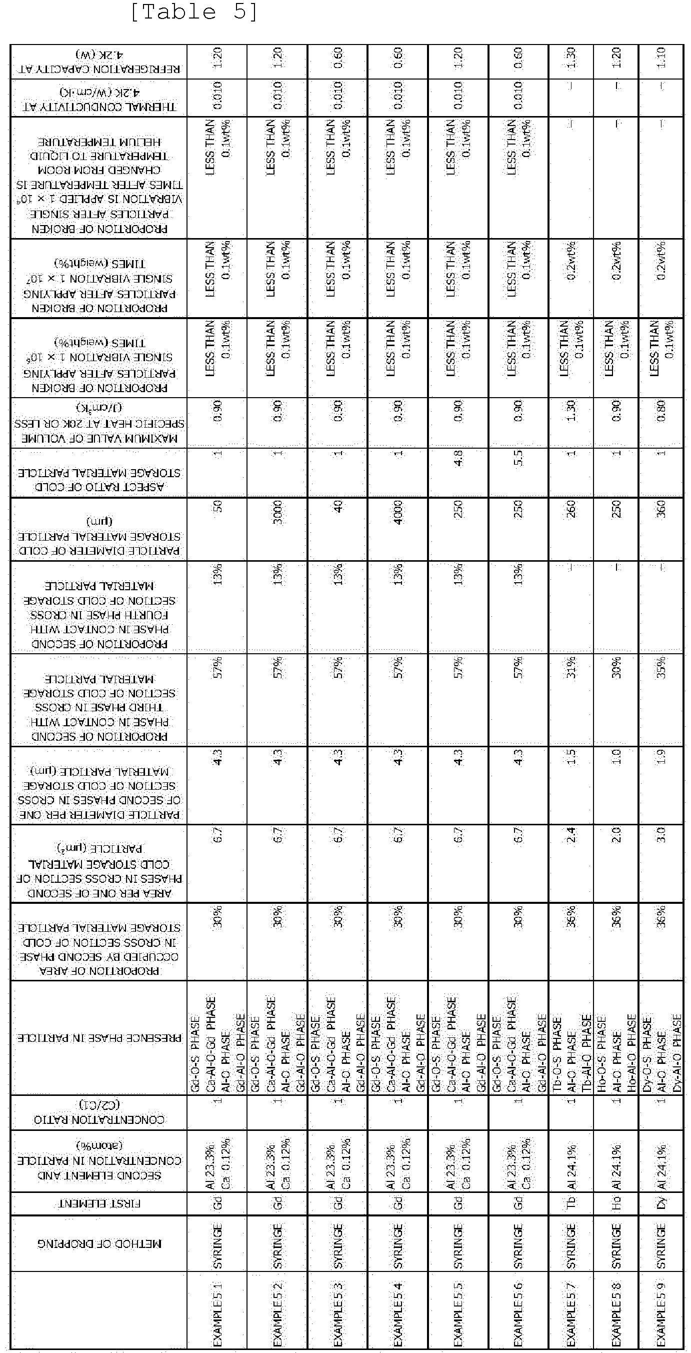

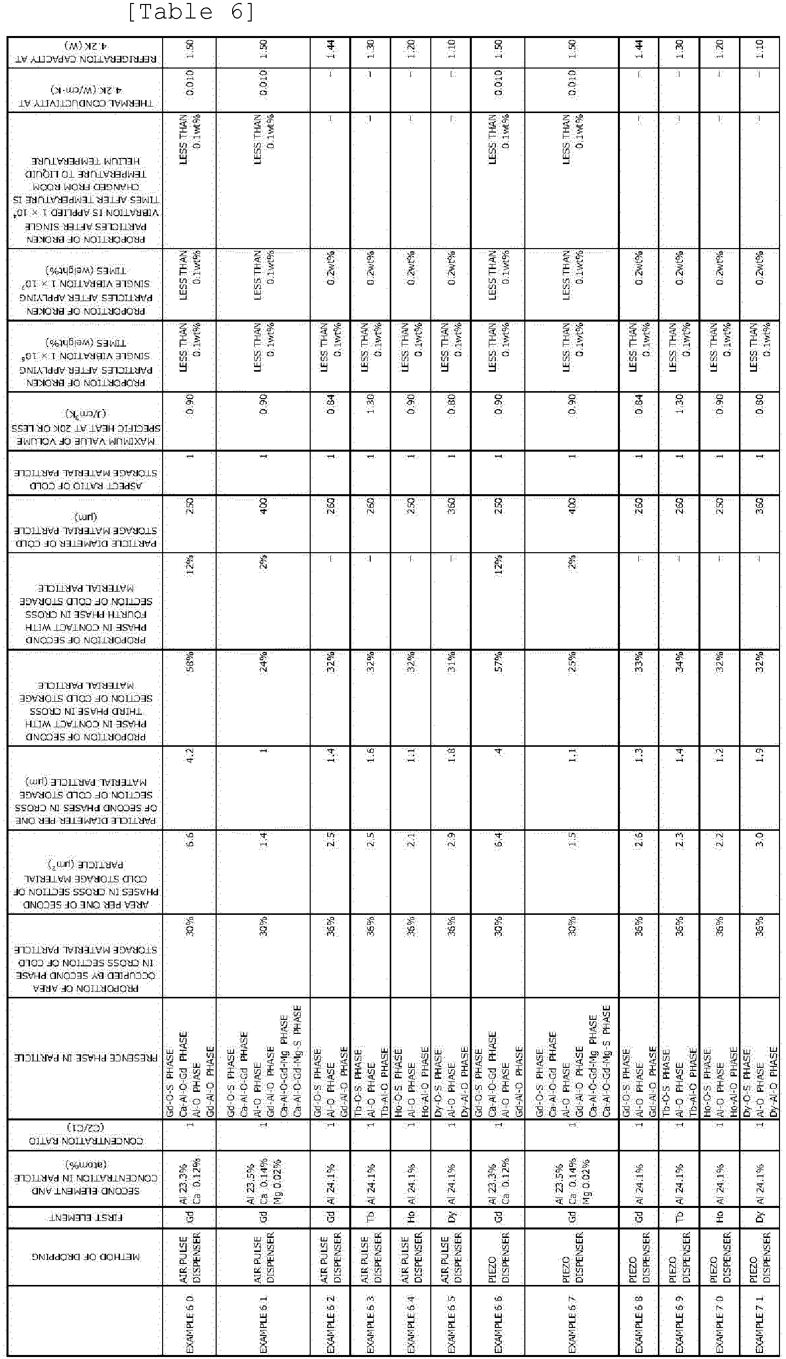

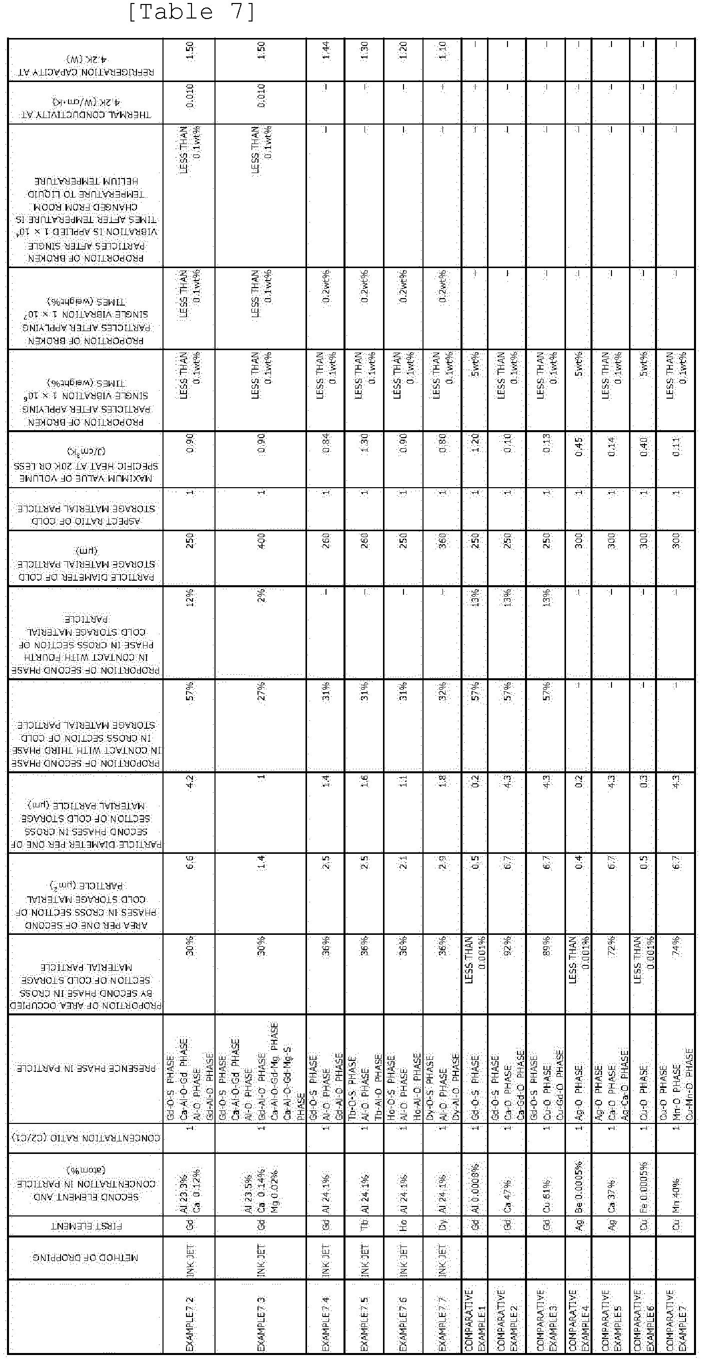

### (Example 1)

Gadolinium oxide powder as a raw material of the cold storage substance was added to an aqueous solution of sodium alginate and mixed to prepare a slurry. Aluminum oxide was also added to the slurry. The mixing time was 12 hours.

The slurry containing the cold storage substance was added dropwise to a calcium lactate aqueous solution as a gelling solution. A syringe was used for dropping the slurry. A diameter of a discharge port of the syringe was 510 um, and a distance from a tip of the syringe to a liquid level of the calcium lactate aqueous solution was 100 mm.

The slurry was held in the gelling solution for 5 hours.

Thereafter, the gelled particles were cleaned with pure water. After cleaning the particles, the particles were dried. After the particles were dried, the particles were sulfurized and sintered.

A heat treatment was performed at 500°C for 4 hours in an atmosphere containing hydrogen sulfide (H₂S) to sulfurize the particles. A heat treatment was performed at 1300°C for 12 hours in an inert gas pressurized atmosphere to sinter the particles.

A cold storage material particle of Example 1 was spherical.

The cold storage substance of the cold storage material particle of Example 1 is gadolinium oxysulfide, the first element is gadolinium (Gd), and the second element is calcium (Ca) and aluminum (Al). The concentration of calcium in the cold storage material particle of Example 1 is 0.12 atom%. The concentration of aluminum in the cold storage material particle is 23.3 atom%.

In the cold storage material particle, a concentration ratio (C2/C1) of the atomic concentration (C2) of the second element in the second region to the atomic concentration (C1) of the second element in the first region was 1. That is, C2/C1 = 1.

In the following Examples and Comparative Examples, a mixing time of the raw material powder and the alginic acid aqueous solution, the retention time (gelation time) of the slurry in the gelling solution, the conditions of the sulfurization heat treatment, the conditions of the sintering heat treatment, and the like are adjusted to be appropriate conditions.

### (Example 2)

A cold storage material particle was produced in the same manner as in Example 1 except that magnesium oxide was used in addition to aluminum oxide.

### (Example 3)

A cold storage material particle was produced in the same manner as in Example 1 except that aluminum chloride was used as the gelling solution.

### (Example 4)

A cold storage material particle was produced in the same manner as in Example 1 except that a magnesium sulfate aqueous solution was used as a gelling solution.

### (Example 5)

A cold storage material particle was produced in the same manner as in Example 1 except that a beryllium sulfate aqueous solution was used as the gelling solution.

### (Example 6)

A cold storage material particle was produced in the same manner as in Example 1 except that a strontium nitrate aqueous solution was used as a gelling solution.

### (Example 7)

A cold storage material particle was produced in the same manner as in Example 1 except that a barium chloride aqueous solution was used as a gelling solution.

### (Example 8)

A cold storage material particle was produced in the same manner as in Example 1 except that a manganese chloride aqueous solution was used as the gelling solution.

### (Example 9)

A cold storage material particle was produced in the same manner as in Example 1 except that an iron chloride aqueous solution was used as the gelling solution.

### (Example 10)

A cold storage material particle was produced in the same manner as in Example 1 except that a copper chloride aqueous solution was used as the gelling solution.

### (Example 11)

A cold storage material particle was produced in the same manner as in Example 1 except that a nickel chloride aqueous solution was used as the gelling solution.

### (Example 12)

A cold storage material particle was produced in the same manner as in Example 1 except that a cobalt chloride aqueous solution was used as the gelling solution.

### (Example 13)

A cold storage material particle was produced in the same manner as in Example 1 except that a part of gadolinium as a cold storage element was replaced with yttrium (Y), the weight of the raw material powder of the cold storage substance in the slurry was increased, and aluminum oxide was not used.

### (Example 14)

A cold storage material particle was produced in the same manner as in Example 1 except that a part of gadolinium as a cold storage element was replaced with cerium (Ce), the weight of the raw material powder of the cold storage substance in the slurry was increased, aluminum oxide was not used, and magnesium chloride was used as a gelling solution.

### (Example 15)

A cold storage material particle was produced in the same manner as in Example 1 except that a part of gadolinium as a cold storage element was replaced with praseodymium (Pr), the weight of the raw material powder of the cold storage substance in the slurry was increased, aluminum oxide was not used, and a beryllium sulfate aqueous solution was used as a gelling solution.

### (Example 16)

A cold storage material particle was produced in the same manner as in Example 1 except that a part of gadolinium as a cold storage element was replaced with neodymium (Nd), the weight of the raw material powder of the cold storage substance in the slurry was increased, aluminum oxide was not used, and a strontium nitrate aqueous solution was used as a gelling solution.

### (Example 17)

A cold storage material particle was produced in the same manner as in Example 1 except that a part of gadolinium as a cold storage element was replaced with promethium (Pm), the weight of the raw material powder of the cold storage substance in the slurry was increased, aluminum oxide was not used, and a barium chloride aqueous solution was used as a gelling solution.

### (Example 18)

A cold storage material particle was produced in the same manner as in Example 1, except that a part of gadolinium as a cold storage element was replaced with samarium (Sm), aluminum oxide was not used, and a manganese chloride aqueous solution was used as a gelling solution.

### (Example 19)

A cold storage material particle was produced in the same manner as in Example 1, except that part of gadolinium as a cold storage element was replaced with europium (Eu), aluminum oxide was not used, and an aluminum chloride aqueous solution was used as a gelling solution.

### (Example 20)

A cold storage material particle was produced in the same manner as in Example 1 except that a part of gadolinium as a cold storage element was replaced with terbium, aluminum oxide was not used, and an iron chloride aqueous solution was used as a gelling solution.

### (Examples 21 to 26)

Cold storage material particles were produced in the same manner as in Example 1 except that a part of gadolinium (Gd) as a cold storage element was replaced with dysprosium (Dy), holmium (Ho), erbium (Er), thulium (Tm), ytterbium (Yb), or lutetium (Lu), the weight of the raw material powder of the cold storage substance in the slurry was reduced, aluminum oxide was not used, and a copper chloride aqueous solution, a nickel chloride aqueous solution, a cobalt chloride aqueous solution, a calcium chloride aqueous solution, or a magnesium chloride aqueous solution was used as a gelling solution.

### (Example 27)

A cold storage material particle of Example 27 is different from that of Example 3 in that the additive metal element is strontium (Sr).

### (Example 28)

A cold storage material particle of Example 28 is different from that of Example 3 in that the concentration of aluminum (Al) in the particle is high.

### (Example 29)

A cold storage material particle of Example 29 is different from the cold storage material particle of Example 1 in that the concentration ratio (C2/C1) of the atomic concentration (C2) of the additive metal element in the second region to the atomic concentration (C1) of the additive metal element in the first region is 1.03.

In the cold storage material particle of Example 29, the gelation time was made shorter than the gelation time in Example 1 by the same producing method as in Example 1.

### (Example 30)

A cold storage material particle of Example 30 is different from the cold storage material particle of Example 29 in that the concentration ratio (C2/C1) of the atomic concentration (C2) of the additive metal element in the second region to the atomic concentration (C1) of the additive metal element in the first region is 1.05.

When the cold storage material particle of Example 30 was produced, the gelation time was shortened as compared with the case of producing the cold storage material particle of Example 29.

### (Example 31)

A cold storage material particle of Example 31 is different from the cold storage material particle of Example 29 in that the concentration ratio (C2/C1) of the atomic concentration (C2) of the additive metal element in the second region to the atomic concentration (C1) of the additive metal element in the first region is 1.1.

When the cold storage material particle of Example 31 was produced, the gelation time was shortened as compared with the case of producing the cold storage material particle of Example 30.

### (Example 32)

A cold storage material particle of Example 32 is different from the cold storage material particle of Example 1 in that silver oxide is contained as the cold storage substance and the cold storage element is silver (Ag). In addition, the cold storage material particle is different from the cold storage material particle of Example 1 in that magnesium (Mg) is contained as the additive metal element and aluminum (Al) is not contained as an additive metal element. The cold storage material particle of Example 32 contains an Ag-O phase and an Ag-Mg-O phase.

### (Example 33)

A cold storage material particle of Example 32 is different from the cold storage material particle of Example 1 in that silver oxide is contained as the cold storage substance and the cold storage element is silver (Ag). The cold storage material particle is different from the cold storage material particle of Example 1 in that aluminum (Al) is not contained as the additive metal element. The cold storage material particle of Example 33 contains an Ag-O phase and an Ag-Ca-O phase.

### (Example 34)

A cold storage material particle of Example 34 is different from the cold storage material particle of Example 1 in that copper oxide is contained as the cold storage substance, and the cold storage element is copper (Cu). The cold storage material particle is different from the cold storage material particle of Example 1 in that strontium (Sr) is contained as the additive metal element and aluminum (Al) is not contained as the additive metal element. The cold storage material particle of Example 34 contains a Cu-O phase and a Cu-Sr-O phase.

### (Example 35)

A cold storage material particle of Example 35 is different from the cold storage material particle of Example 1 in that copper oxide is contained as the cold storage substance, and the cold storage element is copper (Cu). The cold storage material particle is different from the cold storage material particle of Example 1 in that barium (Ba) is contained as the additive metal element and aluminum (Al) is not contained as the additive metal element. The cold storage material particle of Example 35 contains a Cu-O phase and a Cu-Ba-O phase.

### (Examples 36 to 41)

Cold storage material particles of Examples 36 to 41 are different from the cold storage material particle of Example 1 in the area and particle diameter per second phase in the cross section of the cold storage material particle. When the cold storage material particles of Example 36 to 41 were produced, the sintering temperature and the sintering time were changed as compared with the case of producing the cold storage material particle of Example 1.

### (Examples 42 to 50)

Cold storage material particles of Example 42 to 50 are different from the cold storage material particle of Example 1 in the proportion of the second phase in contact with the third phase and the proportion of the second phase in contact with the fourth phase in the cross section of the cold storage material particle. When the cold storage material particles of Examples 42 to 50 were produced, the mixing time of the raw material powder and the alginic acid aqueous solution was changed as compared with the case of producing the cold storage material particle of Example 1.

### (Examples 51 to 56)

Cold storage material particles of Examples 51 to 56 are different from the cold storage material particle of Example 1 in the particle diameter or aspect ratio of the cold storage material particle. When the cold storage material particles of Examples 51 to 56 were produced, the diameter of the discharge port of the syringe and the distance from the tip of the syringe to the surface of the gelling solution were changed as compared with the case of producing the cold storage material particle of Example 1.

### (Examples 57 to 59)

Cold storage material particles were produced in the same manner as in Example 3 except that terbium oxide, holmium oxide, and dysprosium oxide were used instead of gadolinium oxide.

### (Example 60)

A cold storage material particle was produced in the same manner as in Example 1 except that an air pulse dispenser was used instead of a syringe as a method of dropping a slurry. The time during which the rod was driven up and down was 10 ms per cycle, the diameter of the discharge port of the nozzle was 510 um, and the distance from the tip of the nozzle to the liquid level of the calcium lactate aqueous solution was 100 mm. At this time, the production speed of the particles is 100 particles/second, and the production speed is improved as compared with the case of dropping the slurry with a syringe.

In the following Examples, the particle diameter of the particles was adjusted by adjusting the mixing time of the raw material powder and the alginic acid aqueous solution, the slurry viscosity, the retention time (gelation time) of the slurry in the gelling solution, the conditions of the sulfurization heat treatment, the conditions of the sintering heat treatment, the time for driving the rod up and down, the liquid feeding pressure, the diameter of the discharge port of the nozzle, the distance from the tip of the nozzle to the liquid level of the calcium lactate aqueous solution, and the like.

### (Example 61)

A cold storage material particle was produced in the same manner as in Example 60, except that magnesium oxide was used in addition to aluminum oxide.

### (Example 62)

A cold storage material particle was produced in the same manner as in Example 60, except that aluminum chloride was used as the gelling solution.

### (Examples 63 to 65)

A cold storage material particle was produced in the same manner as in Example 60 except that terbium oxide, holmium oxide, and dysprosium oxide were used instead of gadolinium oxide.

### (Example 66)

A cold storage material particle was produced in the same manner as in Example 1 except that a piezo dispenser was used instead of a syringe as a method of dropping a slurry. The time during which the rod was driven up and down was 10 ms per cycle, the diameter of the discharge port of the nozzle was 510 um, and the distance from the tip of the nozzle to the liquid level of the calcium lactate aqueous solution was 100 mm. At this time, the production speed of the particles is 100 particles/second, and the production speed is improved as compared with the case of dropping the slurry with a syringe.

In the following Examples, the particle diameter of the particle was adjusted by adjusting the mixing time of the raw material powder and the alginic acid aqueous solution, the slurry viscosity, the retention time (gelation time) of the slurry in the gelling solution, the conditions of the sulfurization heat treatment, the conditions of the sintering heat treatment, the time during which the rod was driven up and down, the liquid feeding pressure, the diameter of the discharge port of the nozzle, the distance from the tip of the nozzle to the liquid level of the calcium lactate aqueous solution, and the like.

### (Example 67)

A cold storage material particle was produced in the same manner as in Example 66 except that magnesium oxide was used in addition to aluminum oxide.

### (Example 68)

A cold storage material particle was produced in the same manner as in Example 66 except that aluminum chloride was used as the gelling solution.

### (Examples 69 to 71)

Cold storage material particles were produced in the same manner as in Example 66 except that terbium oxide, holmium oxide, and dysprosium oxide were used instead of gadolinium oxide.

### (Example 72)

A cold storage material particle was produced in the same manner as in Example 1 except that a continuous type inkjet was used instead of a syringe as a method of dropping a slurry. The production speed of the particles was set to 500 particles/second by appropriately controlling the waveform of the pressure wave applied to the liquid column. This production speed is improved as compared with the production speed at the time of dropping with a syringe. The diameter of the discharge port of the nozzle was 510 um, and the distance from the tip of the nozzle to the liquid level of the calcium lactate aqueous solution was 100 mm.

In the following Examples, the particle diameter of the particles was adjusted by adjusting the mixing time of the raw material powder and the alginic acid aqueous solution, the slurry viscosity, the retention time (gelation time) of the slurry in the gelling solution, the conditions of the sulfurization heat treatment, the conditions of the sintering heat treatment, the production speed of the particles, the liquid feeding pressure, the diameter of the discharge port of the nozzle, the distance from the tip of the nozzle to the liquid level of the calcium lactate aqueous solution, and the like.

### (Example 73)

A cold storage material particle was produced in the same manner as in Example 72 except that magnesium oxide was used in addition to aluminum oxide.

### (Example 74)

A cold storage material particle was produced in the same manner as in Example 72 except that aluminum chloride was used as the gelling solution.

### (Examples 75 to 77)

Cold storage material particles were produced in the same manner as in Example 72 except that terbium oxide, holmium oxide, and dysprosium oxide were used instead of gadolinium oxide.

### (Comparative Example 1)

A cold storage material particle of Comparative Example 1 is different from the cold storage material particle of Example 3 in that the atomic concentration of aluminum (Al) in the particle is as small as 0.0008 atom%. When the cold storage material particle of Comparative Example 1 was produced, the weight of the raw material powder of the cold storage substance in the slurry was increased as compared with the case of producing the cold storage material particle of Example 3, and aluminum oxide was not used for the slurry.

### (Comparative Example 2)

A cold storage material particle of Comparative Example 2 is different from the cold storage material particle of Example 3 in that aluminum (Al) is not contained and the atomic concentration of calcium (Ca) in the particle is as large as 47 atom%. When the cold storage material particle of Comparative Example 2 was produced, the weight of the raw material powder of the cold storage substance in the slurry was reduced as compared with the case of producing the cold storage material particle of Example 1, and aluminum oxide was not used for the slurry.

### (Comparative Example 3)

The cold storage material particle of Comparative Example 3 is different from the cold storage material particle of Example 1 in that aluminum (Al) is not contained and the atomic concentration of calcium (Ca) in the particle is as large as 61 atom%. When the cold storage material particle of Comparative Example 3 was produced, the weight of the raw material powder of the cold storage substance in the slurry was reduced as compared with the case of producing the cold storage material particle of Example 1, aluminum oxide was not used for the slurry, and a copper chloride aqueous solution was used as a gelling solution.

### (Comparative Example 4)

The cold storage material particle of Comparative Example 4 is different from the cold storage material particle of Example 1 in that it does not contain aluminum (Al) and the atomic concentration of beryllium (Be) in the particle is as low as 0.0005 atom%. When the cold storage material particle of Comparative Example 4 was produced, the weight of the raw material powder of the cold storage substance in the slurry was increased as compared with the case of producing the cold storage material particle of Example 32, and the beryllium sulfate aqueous solution was used as the gelling solution.

### (Comparative Example 5)

The cold storage material particle of Comparative Example 5 is different from the cold storage material particle of Example 33 in that the atomic concentration of calcium (Ca) in the particle is as large as 37 atom%. When the cold storage material particle of Comparative Example 5 was produced, the weight of the raw material powder of the cold storage substance in the slurry was reduced as compared with the case of producing the cold storage material particle of Example 33.

### (Comparative Example 6)

The cold storage material particle of Comparative Example 6 is different from the cold storage material particle of Example 1 in that aluminum (Al) is not contained and the atomic concentration of iron (Fe) in the particle is as small as 0.0005 atom%. When the cold storage material particle of Comparative Example 6 was produced, the weight of the raw material powder of the cold storage substance in the slurry was increased as compared with the case of producing the cold storage material particle of Example 34, and an iron chloride aqueous solution was used as a gelling solution.

### (Comparative Example 7)

The cold storage material particle of Comparative Example 7 is different from the cold storage material particle of Example 35 in that the atomic concentration of manganese (Mn) in the particle is as large as 40 atom%. When the cold storage material particle of Comparative Example 7 was produced, the weight of the raw material powder of the cold storage substance in the slurry was reduced as compared with the case of producing the cold storage material particle of Example 35, and the manganese chloride aqueous solution was used as the gelling solution.

The maximum value of the volume specific heat at 20K or less was measured for each of the produced cold storage material particles. The results are illustrated in Tables 1 to 7.

The mechanical strength required for the cold storage material particles was evaluated when the cold storage device constituting the refrigerator was filled with the cold storage material particles and the refrigerator was operated. A cylindrical container having a diameter of 15 mm and a height of 5 mm was filled with each of the produced cold storage material particles. A sufficient amount of cold storage material particles was filled so that the cold storage material particles were fixed in a cylindrical container and did not move freely. A single vibration having an amplitude of 2 mm and a maximum acceleration of 400 m/s² was applied to the container 1 × 10⁶ times and 1 × 10⁷ times. As a result, the proportion of the broken cold storage material particles is illustrated in Tables 1 to 7.

The mechanical strength required for the cold storage material particles was evaluated when the cold storage device constituting the refrigerator was filled with the cold storage material particles and the refrigerator was operated. In addition, resistance to a temperature change, which is required for the cold storage material particles when the temperature of the cold storage material goes back and forth between room temperature and cryogenic temperature before and after the operation of the refrigerator, was also evaluated at the same time. A cylindrical container having a diameter of 15 mm and a height of 5 mm was filled with each of the produced cold storage material particles. A sufficient amount of cold storage material particles was filled so that the cold storage material particles were fixed in a cylindrical container and did not move freely. After filling, the cylindrical container was cooled from room temperature to liquid helium temperature and returned to room temperature again. Next, a single vibration having an amplitude of 2 mm and a maximum acceleration of 400 m/s² was applied to the container 1 × 10⁴ times. As a result, the proportion of the broken cold storage material particles is illustrated in the table.

The thermal conductivity and the refrigeration capacity of the cold storage material particle at 4.2K are illustrated in the table. The refrigeration capacity was examined by a two-stage GM refrigerator having a power consumption of 3.4 kW. A Cu mesh was housed in the first cold storage device, the first layer was filled with lead (Pb) as a cold storage substance on the high-temperature side of the second cold storage device, the second layer was filled with HoCu₂ as a cold storage substance, and the third layer on the low-temperature side was filled with cold storage material particles to be evaluated.

As in Comparative Example 3, it is found that when the atomic concentration of the additive metal element in the cold storage material particle is more than 60 atom%, the maximum value of the volume specific heat is less than 0.3. This is considered to be because the proportion of the cold storage substance in the cold storage material particles decreased as the proportion of the additive metal element in the cold storage material particles increased.

As in Comparative Example 1, Comparative Example 4, and Comparative Example 6, when the atomic concentration of the additive metal element in the cold storage material particle becomes less than 0.001 atom%, the proportion of the destroyed cold storage material particle increases to 5 wt% or more. This is considered to be because the sintering of the cold storage material particles does not sufficiently proceed due to the low atomic concentration of the additive metal element.

The proportion of broken particles when a single vibration is applied 1 × 10⁷ times is compared among Examples 1 to 12. The proportion of the broken particles in Examples 1 and 2 containing calcium (Ca) as the additive metal element is low. This is considered to be because calcium (Ca) has a high ability to particularly promote sintering of the cold storage material particles.

As in Examples 29 to 31, when the concentration ratio (C2/C1) of the atomic concentration (C2) of the additive metal element in the second region to the atomic concentration (C1) of the additive metal element in the first region is larger than 1, the maximum value of the volume specific heat increases. This is considered to be because the proportion occupied by the cold storage substance increases in the central portion of the cold storage material particle.

From Tables 1 to 7, when the proportion of the area occupied by the second phase in the cross section of the cold storage material particle exceeds 0.001%, the proportion of the destroyed cold storage material particle rapidly decreases. It can be seen that when the proportion of the area occupied by the second phase exceeds 0.001%, the mechanical strength is improved.

Meanwhile, when the proportion of the area occupied by the second phase in the cross section of the cold storage material particle is less than 75%, it is illustrated that the maximum value of the volume specific heat of 20K or less is improved. By controlling the proportion of the area occupied by the second phase in this manner, it is possible to provide a cold storage material particle having high thermal conductivity and high strength.

Tables 1 to 7 illustrate that when the area per one of the second phases in the cross section of the cold storage material particle exceeds 0.001 µm², the thermal conductivity is remarkably improved. Meanwhile, when the area per one of the second phases in the cross section of the cold storage material particle is less than 6000 µm², the proportion of the broken cold storage material particle after applying the single vibration 1 × 10⁷ times is remarkably decreased. It is found that when the area per one of the second phases in the cross section of the cold storage material particle is less than 6000 µm², the mechanical strength of the cold storage material particle is improved. By controlling the area of each phase in this manner, it is possible to provide a cold storage material particle having high thermal conductivity and high strength.

From Tables 1 to 7, it can be seen that the thermal conductivity is remarkably improved when the particle diameter per one of the second phases in the cross section of the cold storage material particle exceeds 0.1 um. Meanwhile, when the particle diameter per one of the second phases in the cross section of the cold storage material particles is less than 100 um, the proportion of the broken cold storage material particles after applying single vibration 1 × 10⁷ times is remarkably decreased. It can be seen that the mechanical strength of the cold storage material particle is improved when the particle diameter per one of the second phases in the cross section of the cold storage material particle is less than 100 um. By controlling the particle diameter of each phase in this manner, it is possible to provide a cold storage material particle having high thermal conductivity and high strength.

As can be seen from Tables 1 to 7, when the proportion of the second phase in contact with the third phase in the cross section of the cold storage material particle exceeds 20%, the proportion of the broken particles decreases after the single vibration is applied 1 × 10⁴ times after the temperature is changed from room temperature to the liquid helium temperature. It can be seen that when the proportion of the second phase in contact with the third phase exceeds 20%, the mechanical strength is improved when a temperature change is applied to the cold storage material particle.

Meanwhile, when the proportion of the second phase in contact with the third phase in the cross section of the cold storage material particle is less than 90%, the proportion of the broken particles decreases after the single vibration is applied 1 × 10⁴ times after the temperature is changed from room temperature to the liquid helium temperature. When the proportion of the second phase in contact with the third phase in the cross section of the cold storage material particle is less than 90%, it is illustrated that the mechanical strength is improved when a temperature change is applied to the cold storage material particle. By controlling the contact proportion between the second phase and the third phase in this manner, the mechanical strength is improved when a temperature change is applied to the cold storage material particle.

From Tables 1 to 7, when the proportion of the second phase in contact with the third phase exceeds the proportion of the second phase in contact with the fourth phase in the cross section of the cold storage material particle, the proportion of the broken particle decreases after the single vibration is applied 1 × 10⁴ times after the temperature is changed from room temperature to the liquid helium temperature. It can be seen that when the proportion of the second phase in contact with the third phase exceeds the proportion of the second phase in contact with the fourth phase in the cross section of the cold storage material particle, the mechanical strength is improved when a temperature change is applied to the cold storage material particle.

Tables 1 to 7 illustrate that when the particle diameter of the cold storage material particle falls within a range of 50 um or more and 3000 um or less, the refrigeration capacity at 4.2K is remarkably improved.

Tables 1 to 7 illustrate that when the aspect ratio of the cold storage material particle is 5 or less, the refrigeration capacity at 4.2K is remarkably improved.

From Examples 60, 66, and 72, it can be seen that the production speed of the cold storage material particle is greatly improved when the dispenser or the inkjet is used as compared with the case of using the syringe.

From Tables 1 to 7, it can be seen that the same particle diameter, aspect ratio, dispersion relation of phases in the particles, strength, and specific heat are exhibited as long as conditions other than the method of dropping the liquid are the same regardless of whether the method of dropping the liquid is a syringe, a dispenser, or an inkjet.

From Tables 1 to 7, it can be seen that the performance and reliability of the refrigerator equipped with the particles are equivalent as long as the particles are equivalent in particle diameter, aspect ratio, dispersion relation of phases in the particles, strength, and specific heat even when the method of dropping the liquid is different.

From the above Examples, the effects exhibited by the cold storage material particles of the first to third embodiments were confirmed.

As described above, in the first to third embodiments, the case where the rare earth element to be the cold storage element is gadolinium (Gd) has been described as an example, but the cold storage element may be a rare earth element other than gadolinium (Gd).

In the first to third embodiments, the case where the additive metal element is calcium (Ca) or aluminum (Al) has been described as an example, but the additive metal element may be magnesium (Mg), beryllium (Be), strontium (Sr), barium (Ba), radium (Ra), manganese (Mn), iron (Fe), copper (Cu), nickel (Ni), or cobalt (Co).

In addition, although the case of an air pulse dispenser or a piezo dispenser has been described as an example of the dispenser, a plunger dispenser may be used.

Furthermore, the case of the continuous type inkjet has been described as an example of the inkjet, but an on-demand type inkjet may be used.

While certain embodiments have been described, these embodiments have been presented by way of example only, and are not intended to limit the scope of the inventions. These embodiments may be embodied in a variety of other forms; furthermore, various omissions, substitutions and changes may be made without departing from the spirit of the inventions. For example, a component of one embodiment may be replaced or changed with a component of another embodiment. These embodiments and modifications thereof are included in the scope and gist of the invention, and are included in the invention described in the claims and the equivalent scope thereof.

### Reference Signs List

10 Cold storage material particle according to first embodiment
20 Cold storage material particle of second embodiment
30 Cold storage material particle according to third embodiment
30a Low-concentration region
30b High-concentration region
100 Cold storage type cryogenic refrigerator
500 Cryopump
600 Superconducting magnet
700 Nuclear magnetic resonance imaging apparatus
800 Nuclear magnetic resonance apparatus
900 magnetic field application type single crystal pulling apparatus

## Claims

1. A cold storage material particle comprising:
at least one first element selected from the group consisting of a rare earth element, silver (Ag), and copper (Cu); and
a second element being different from the first element and forming a multivalent metal ion in an aqueous solution,
wherein an atomic concentration of the second element is 0.001 atomic% or more and 60 atomic% or less, and
a maximum value of volume specific heat at a temperature of 20K or less is 0.3 J/cm³·K or more.

2. The cold storage material particle according to claim 1, wherein the second element is at least one element selected from the group consisting of beryllium (Be), magnesium (Mg), calcium (Ca), strontium (Sr), barium (Ba), radium (Ra), manganese (Mn), aluminum (Al), iron (Fe), copper (Cu), nickel (Ni), and cobalt (Co).

3. The cold storage material particle according to claim 1 or 2, further comprising oxygen.

4. The cold storage material particle according to any one of claims 1 to 3, wherein the second element contains two or more different elements.

5. The cold storage material particle according to any one of claims 1 to 4, further comprising:
a first phase containing the first element; and
a second phase containing the second element and
being different from the first phase,
wherein an atomic concentration of the second element in the second phase is larger than an atomic concentration of the second element in the first phase.

6. The cold storage material particle according to claim 5, further comprising a third phase containing the first element and the second element and being different from the first phase and the second phase.

7. The cold storage material particle according to claim 5,
wherein the second element includes two or more different elements including an element α and an element β,
the second phase contains the element α and the element β,
the cold storage material particle further comprising:
a third phase containing the element β and being different from the first phase and the second phase; and
a fourth phase containing the first element and the element β and being different from the first phase, the second phase, and the third phase.

8. The cold storage material particle according to claim 7, wherein a proportion of the second phase in contact with the third phase among the second phase is higher than a proportion of the second phase in contact with the fourth phase among the second phase.

9. The cold storage material particle according to any one of claims 6 to 8, wherein 20% or more and 90% or less of the second phase is in contact with the third phase.

10. The cold storage material particle according to any one of claims 5 to 9, wherein a proportion of an area occupied by the second phase is 0.001% or more and 75% or less in a cross section of the cold storage material particle.

11. The cold storage material particle according to any one of claims 5 to 10, wherein an area per one of the second phases is 0.001 µm² or more and 6000 µm² or less in a cross section of the cold storage material particle.

12. The cold storage material particle according to any one of claims 5 to 11, wherein a particle diameter per one of the second phases is 0.1 um or more and 100 um or less in a cross section of the cold storage material particle.

13. The cold storage material particle according to any one of claims 1 to 12, further comprising:
a first region; and
a second region being closer to an outer edge of the particle than the first region and having a higher atomic concentration of the second element than the first region.

14. The cold storage material particle according to any one of claims 1 to 13
wherein the first element contains gadolinium (Gd), and the second element contains at least one element selected from the group consisting of beryllium (Be), magnesium (Mg), calcium (Ca), strontium (Sr), barium (Ba), and radium (Ra), and aluminum (Al),
the cold storage material particle further comprising sulfur (S).

15. The cold storage material particle according to any one of claims 1 to 14, further comprising an oxide of the first element or an oxysulfide of the first element.

16. The cold storage material particle according to claim 15, further comprising silver oxide, copper oxide, or gadolinium oxysulfide.

17. The cold storage material particle according to any one of claims 1 to 16, wherein a particle diameter is 50 um or more and 3 mm or less.

18. The cold storage material particle according to any one of claims 1 to 17, wherein an aspect ratio is 5 or less.

19. A cold storage device filled with a plurality of the cold storage material particles according to any one of claims 1 to 18.

20. A refrigerator comprising the cold storage device according to claim 19.

21. A cryopump comprising the refrigerator according to claim 20.

22. A superconducting magnet comprising the refrigerator according to claim 20.

23. A nuclear magnetic resonance imaging apparatus comprising the refrigerator according to claim 20.

24. A nuclear magnetic resonance apparatus comprising the refrigerator according to claim 20.

25. A magnetic field application type single crystal pulling apparatus, comprising the refrigerator according to claim 20.

26. A method for producing a cold storage material particle, the method comprising:
forming a slurry by mixing powder containing at least one first element selected from the group consisting of a rare earth element, silver (Ag), and copper (Cu) with an alginic acid aqueous solution;
discharging the slurry in the form of droplets into a gelling solution containing a second element forming multivalent metal ions;
forming particles being gelled by holding the slurry in the gelling solution; and
sintering the particles.

27. The method for producing a cold storage material particle according to claim 26,
further comprising, after the forming the particles and before the sintering the particles:
cleaning the particles; and
drying the particles.

28. The method for producing a cold storage material particle according to claim 27,
further comprising, after the drying the particles and before the sintering the particles:
performing heat treatment on the particles in a sulfurization atmosphere.

29. The method for producing a cold storage material particle according to any one of claims 26 to 28,
wherein in the discharging the slurry into the gelling solution, any one device of an air pulse dispenser, a plunger dispenser, a piezo dispenser, an inkjet, a pipette, and a syringe is used.

30. The method for producing a cold storage material particle according to claim 29, wherein a diameter of a discharge port of the any one of the devices is 50 um or more and 3000 um or less.
